# EUROPEAN PATENT APPLICATION

(11) **EP 1 676 841 A1**
(43) Date of publication of application: **05.07.2006**
(21) Application number: 04380290.9
(22) Date of filing: 30.12.2004
(51) Int. Cl.: C07D 231/56, C07D 401/04, C07D 409/12, C07D 409/14, C07D 513/04, A61K 31/416, A61K 31/429, A61K 31/454, A61P 3/00, A61P 25/00

(54) **Substitited indazolyl sulfonamide and 2,3-dihydro-indolyl sulfonamide compounds, their prepartion and use in medicaments**

(71) Applicant: Esteve Laboratorios Dr. Esteve S.A., 08041 Barcelona (ES)
(72) Inventor: Merce-Vidal, Ramón, 08021 Barcelona (ES); Codony Soler, Xavier, 08301 Mataró (Barcelona) (ES); Dordal-Zueras, 08016 Barcelona (ES)
(74) Representative: Carvajal y Urquijo, Isabel

(57) **Abstract**

The present invention relates to substituted indazolyl sulfonamide and 2,3-dihydro-indolyl sulfonamide compounds of general formula I, 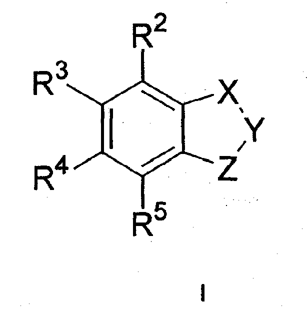 a process for their preparation, medicaments comprising said substituted indazolyl sulfonamide and 2,3-dihydro-indolyl sulfonamide compounds as well as the use of said substituted indazolyl sulfonamide and 2,3-dihydro-indolyl sulfonamide compounds for the preparation of medicaments, which are particularly suitable for the prophylaxis and/or treatment of disorders or diseases that are at least partially mediated via 5-HT₆ receptors.

## Description

The present invention relates to substituted indazolyl sulfonamide and 2,3-dihydro-indolyl sulfonamide compounds of general formula I, 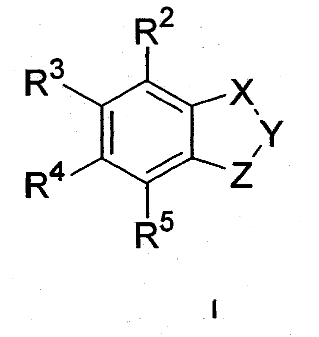 a process for their preparation, medicaments comprising said substituted indazolyl sulfonamide and 2,3-dihydro-indolyl sulfonamide compounds as well as the use of said substituted indazolyl sulfonamide and 2,3-dihydro-indolyl sulfonamide compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol., 1998, 125, 1562; D.C. Rogers et al., Br. J. Pharmacol. Suppl., 1999,127, 22P; A. Bourson et al., J. Pharmacol. Exp. Ther., 1995, 274, 173; A.J. Sleight, et al., Behav. Brain Res. , 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther., 1994, 268, 1403; C.E. Glatt et al., Mol. Med. , 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol., 2000, 130, 1597; C. Gérard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the substituted indazolyl sulfonamide and 2,3-dihydro-indolyl sulfonamide compounds of general formula I, Ia, Ib, Ic or Id given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one aspect the present invention relates to a substituted indazolyl sulfonamide or 2,3-dihydro-indolyl sulfonamide compound of general formula I, 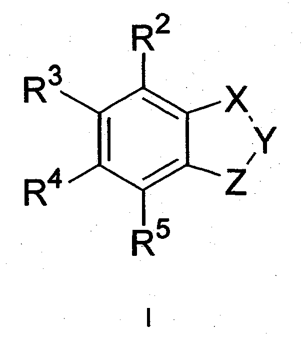 wherein
X-Y from left to right represents CR¹=N and Z is N[(CH₂)ₙR⁶]
or
X-Y from left to right represents CR⁷=N, Z is NH, R⁷ represents the following moiety 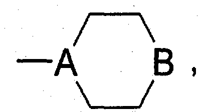 A represents CH or N and B represents NR⁸, 0 or S;
X-Y from left to right represents C[(CH₂)ₙR⁹]=N and Z is NR¹⁰
or
X-Y- represents CH₂-CH₂ and Z is N[(CH₂)ₙR¹¹];
n is 0, 1, 2, 3 or 4;
R¹ represents a hydrogen atom; NO₂; -NH₂; -SH; -OH; -CN; -C(=O)-R¹²; -OR¹³; - SR¹⁴; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -SH, -O-CH₃, -O-C₂H₅, -NO₂, -CN and -S-CH₃; or a 5- to 14-membered aryl or heteroaryl radical, which may be substituted with 1, 2 or 3 substituent(s) independently selected from the group consisting of -CF₃, C₁₋₅₋alkyl, -O-C₁₋₅-alkyl, -S-C₁₋₅-alkyl, -C(=O)-OH, -C(=O)-O-C₁₋₅-alkyl, -O-O(=O)-C₁₋₅₋alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
R², R³, R⁴ and R⁵, independent from one another, each represent a hydrogen atom; - NO₂; -NH₂; -SH; -OH; -CN; -C(=O)-H; -C(=O)-R¹²; -OR¹³; -SR¹⁴; -N(R¹⁵)-S(=O)₂-R¹⁶; - NH-R¹⁷; -NR¹⁸R¹⁹; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH2, -SH, -O-CH₃, -O-C₂H₅, - NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH,-C(=O)-O-C₁₋₅-alkyl, -O-C(=O)-C₁₋₅-alkyl, F, Cl, Br, I, -CN, -OCF₃, -SCF₃, -OH, -SH, - NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅₋alkyl, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
with the proviso that at least one of the substituents R², R³, R⁴ and R⁵ represents a - N(R¹⁵)-S(=O)₂-R¹⁶ moiety;
R⁶, R⁹ and R¹¹, independent from one another, each represent a -NR²⁰R²¹ radical
or
a saturated or unsaturated 3-, 4-, 5-, 6-, 5- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), - C(=O)-OH, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) as ring member(s) independently selected from the group consisting of nitrogen, oxygen and sulfur;
R⁸ represents -C(=O)-R²²; a linear or branched, saturated or unsaturated C₁₋₁₀ aliphatic radical which may be substituted with 1, 2 or 3 substituent(s) independently selected from the group consisting of F, Cl, Br, -OH, -NH2, -SH, -O-CH₃, -O-C₂H₅,-NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH,-C(=O)-O-C₁₋₅-alkyl, -O-C(=O)-C₁₋₅-alkyl, F, Cl, Br, I, -CN, OCF₃, -SCF₃, -OH, -SH, - NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅₋alkyl, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which maybe bonded via a linear or branched C₁₋₆ alkylene, C₂₋₆ alkenylene or C₂₋₆ alkinylene group which maybe substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and - S-CH₃ and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, -S-C₁₋₅₋alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and wherein the heteroaryl radical contains 1, 2 or 3 heteroatom(s) independently selected from the group consisting of nitrogen, oxygen and sulfur as ring member(s);
R¹⁰ represents a hydrogen atom; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, =O=CH₃, -O-C₂H₅,-NO₂, -CN, -NH-CH₃ and -S-CH₃; or a -S(=O)₂-R²³ moiety;
R¹², R¹³, R¹⁴, R¹⁷, R¹⁸ and R¹⁹, independent from one another, each represent a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and - S-CH₃; a saturated or unsaturated 3-, 4-, 5-, 6-, 5- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be bonded via a linear or branched C₁₋₆ alkylene group; or a 5- to 14-membered aryl or heteroaryl radical, which may be substituted with 1, 2 or 3 substituent(s) independently selected from the group consisting of -CF₃, C₁₋₅-alkyl, - O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulfur as ring member(s);
R¹⁵ represents a hydrogen atom; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, - NO₂, -CN, -NH-CH₃ and -S-CH₃; ora -S(=O)₂-R²⁴ moiety;
R¹⁶ and R²⁴, independent from one another, each represent a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, - S-C₁₋₅-alkyl, -C(=O)-OH, -C(=O)-O-C₁₋₅-alkyl, -O-C(=O)-C₁₋₅-alkyl, F, Cl, Br, I, -CN, - OCF₃, -SCF₃, -OH, -SH, -NH₂, -NH(C₁₋₅-alkyl), -N(C₁₋₅-alkyl)₂, -NH-C(=O)-C₁₋₅-alkyl, - N(C₁₋₅-alkyl)-C(=O)-C₁₋₅-alkyl, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, - NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
R²⁰ and R²¹, independent from one another, each represent a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and - S-CH₃;
or
R²⁰ and R²¹ together with the bridging nitrogen form a saturated, unsaturated or aromatic 3- to 9-membered heterocyclic ring which may be substituted with 1, 2 or 3 substituent(s) independently selected from the group consisting of C₁₋₅-alkyl, -O-C₁₋₅₋alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, =SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅₋alkyl, -O-C₁₋₅-alkyl, -S-C₁₋₅-alkyl, oxo (=O); thioxo (=S), -C(=O)-OH, -C(=O)-O-C₁₋₅₋alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1,2 or 3 heteroatom(s) independently selected from the group consisting of nitrogen, oxygen and sulfur; R²² represents a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH, -C(=O)-O-C₁₋₅₋alkyl, -O-C(=O)-C₁₋₅-alkyl, F, Cl, Br, I, -CN, -OCF₃, -SCF₃, -OH, -SH, -NH₂, -NH(C₁₋₅₋alkyl), -N(C₁₋₅-alkyl)₂, -NH-C(=O)-C₁₋₅-alkyl, -N(C₁₋₅-alkyl)-C(=O)-C₁₋₅-alkyl, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s)
and
R²³ represents a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; a saturated or unsaturated 3-, 4-, 5-, 6-, 5- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅₋alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be bonded via a linear or branched C₁₋₆ alkylene group; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH, -C(=O)-O-C₁₋₅₋alkyl, -O-C(=O)-C₁₋₅-alkyl, F, Cl, Br, I, -CN, -OCF₃, -SCF₃, -OH, -SH, -NH₂, -NH(C₁₋₅₋alkyl), -N(C₁₋₅-alkyl)₂, -NH-C(=O)-C₁₋₅-alkyl, -N(C₁₋₅-alkyl)-C(=O)-C₁₋₅-alkyl, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅₋alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl),C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1; 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, -NH₂, -SH, -O-CH₃, -O-C₂H₅, - NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

### Preferred is a compound of general formula I, wherein

X-Y from left to right represents CR¹=N and Z is N[(CH₂)ₙR⁶]
or
X-Y from left to right represents CR⁷=N, Z is NH, R⁷ represents the following moiety 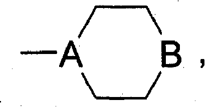 and A represents CH and B represents NR⁸
or
A represents N and B represents NR⁸
or
A represents N and B represents O
or
A represents N and B represents S
or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1, 2, 3 or 4;
R¹ represents a hydrogen atom; NO₂; -NH₂; -SH; -OH; -CN; -C(=O)-R¹²; -OR¹³; -SR¹⁴; F; Cl, Br; I; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl; pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which maybe bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -0-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂₋phenyl, cyclopropyl, cyclobutyl, cyclopentyl, cyclohexyl, phenyl, phenoxy and benzyl;
R², R³, R⁴ and R⁵, independent from one another, each represent a hydrogen atom; - NO₂; -NH₂; -SH; -OH; -CN; -C(=O)-H; -C(=O)-R¹²; -OR¹³; -SR¹⁴; -N(R¹⁵)-S(=O)₂-R¹⁶; - NH-R¹⁷; -NR¹⁸R¹⁹; F; Cl; Br; I; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -0-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂₋CH₃, -C(=O)-O-CH(CH₃)₂, -C(=O)-O-C(CH₃)₃, -O-C(=O)-CH₃, -O-C(=O)-CH₂-CH₃, - O-C(=O)-CH(CH₃)₂, -O-C(=O)-C(CH₃)₃, F, Cl, Br, I, -CN, -OCF₃, -SCF₃, -OH, -SH, - NH₂, -NH-CH₃, -NH-C₂H₅, -NH-CH(CH₃)₂, -NH-C(CH₃)₃, -N(CH₃)₂, -N(C₂H₅)₂, - N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
with the proviso that at least one of the substituents R², R³, R⁴ and R⁵ represents a - N(R¹⁵)-S(=O)₂-R¹⁶ moiety;
R⁶, R⁹ and R¹¹, independent from one another, each represent a -NR²⁰R²¹ radical
or
a (hetero)cycloaliphatic radical selected from the group consisting of 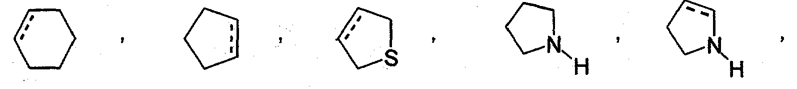 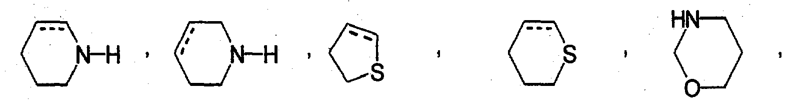 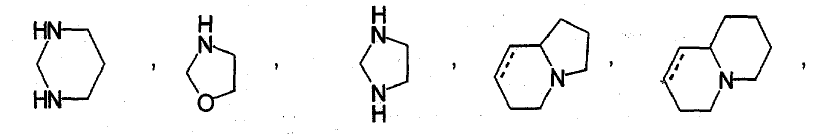 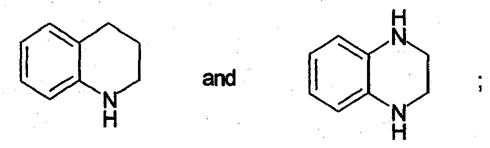 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-CH₂-CH₃, -C(=O)-O-CH(CH₃)₂, -C(=O)-O-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-O(CH₃)₃, -C(=O)-N(CH₃)₂, - C(=O)-N(C₂H₅)₂, -S(=O)₂-CH₃, -S(=O)₂-C₂H₅ and -S(=O)₂-phenyl in any position including the -NH groups and is not bonded via a nitrogen atom and, if present, the dotted line represents an optional chemical bond;
R⁸ represents -C(=O)-R²²; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-prflpyl, isopropyl, n-butyl, sec-butyl, isobutyl, tert-butyl, n-pentyl, sec-pentyl, neo-pentyl, -0-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂₋CH₃, -C(=O)-O-CH(CH₃)₂, -C(=O)-O-C(CH₃)₃, -O-O(=O)-CH₃, -O-C(=O)-CH₂-CH₃, - O-C(=O)-CH(CH₃)₂, -O-C(=O)-C(CH₃)₃, F, Cl, Br, I, -CN, -OCF₃, -SCF3, -OH, -SH, - NH₂, -NH-CH₃, -NH-C₂H₅, -NH-CH(CH₃)₂, -NH-C(CH₃)₃, -N(CH₃)₂, -N(C₂H₅)₂, - N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
R¹⁰ represents a hydrogen atom; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O=CH₃, -O-C₂H₅, -NO₂, -CN and -S-CH₃ or a -S(=O)₂-R²³ moiety;
R¹², R¹³, R¹⁴, R¹⁷, R¹⁸ and R¹⁹, independent from one another, each represent an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, - NH₂, -SH, -O-CH₃, -O-C₂H₅, -NO₂, -CN and -S-CH₃; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂₋CH₃, -S-CH(CH₃)₂, -S-C(CH₃)₃, -C(=O)-OH, -C(=O)-O-CH₃, -C(=O)-O-CH₂-CH₃, - C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, - N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂₋C₂H₅ and -S(=O)₂-phenyl; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂₋CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, - C(=O)-O-C(CH₃)₃, -O-C(=O)-CH₃, -O-C(=O)-CH₂-CH₃, -O-C(=O)-CH(CH₃)₂, -0-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, - CF₂H, -CFH₂, -C(=O)-NH₂, -C(=O)-NH-CH₃, -C(=O)-NH-C₂H₅, -C(=O)-NH-C(CH₃)₃, - C(=O)-N(CH₃)₂, -C(=O)-N(C₂H₅)₂, -S(=O)₂-CH₃, -S(=O)₂-C₂H₅, -S(=O)₂-phenyl, cyclopropyl, cyclobutyl, cyclopentyl, cyclohexyl, phenyl, phenoxy and benzyl;
R¹⁵ represents a hydrogen atom; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or a -S(=O)₂-R²⁴ moiety;
R¹⁶ and R²⁴, independent from one another, each represent an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂phenyl, cyclopropyl, cyclobutyl, cyclopentyl, cyclohexyl, phenyl, phenoxy and benzyl;
R²⁰ and R²¹, independent from one another, each represent a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, - NH₂, -SH, -O-CH₃, -O-C₂H₅, -NO₂, -CN and -S-CH₃
or
R²⁰ and R²¹ together with the bridging nitrogen atom form a moiety selected from the group consisting of 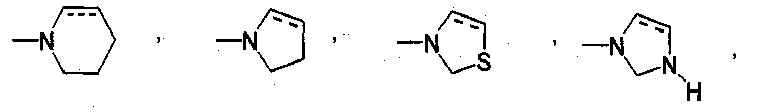 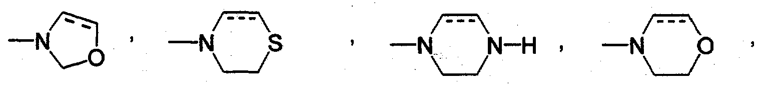 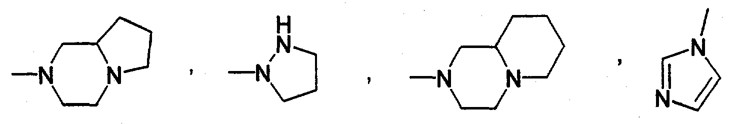 and 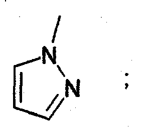 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -C-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-CH₂-CH₃, -C(=O)-O-CH(CH₃)₂, -C(=O)-O-C(CH₃)₃, -0-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, - C(=O)-N(C₂H₅)₂, -S(=O)₂-CH₃, -S(=O)₂-C₂H₅ and -S(=O)₂-phenyl in any position including the -NH groups; and, if present, the dotted line represents an optional chemical bond;
R²² represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂₋phenyl, cyclopropyl, cyclobutyl, cyclopentyl, cyclohexyl, phenyl, phenoxy and benzyl;
and
R²³ represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-CH₂-CH₃, -C(=O)-O-CH(CH₃)₂, -C(=O)-O-C(CH₃)₃, -0-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, - C(=O)-N(C₂H₅)₂, -S(=O)₂-CH₃, -S(=O)₂-C₂H₅ and -S(=O)₂-phenyl; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)=CH₃, -O=C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂₋phenyl, cyclopropyl, cyclobutyl, cyclopentyl, cyclohexyl, phenyl, phenoxy and benzyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred is a compound of general formula I, wherein
X-Y from left to right represents CR¹=N and Z is N[(CH₂)ₙR⁶]
or
X-Y from left to right represents CR⁷=N, Z is NH, R⁷ represents the following moiety 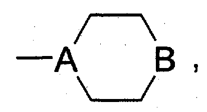 and A represents CH and B represents NR⁸
or
A represents N and B represents NR⁸
or
A represents N and B represents 0
or
A represents N and B represents S
or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1 or 2;
R¹ represents a hydrogen atom; NO₂; -NH₂; -SH; -OH; -CN; F; Cl, Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, - NH₂, -SH, -O-CH₃, -O-C₂H₅, -NO₂, -CN and -S-CH₃;
R², R³, R⁴ and R⁵, independent from one another, each represent a hydrogen atom; - NO₂; -NH₂; -SH; -OH; -CN; -C(=O)-H; -C(=O)-R¹²; -OR¹³; -SR¹⁴; -N(R¹⁵)-S(=O)₂-R¹⁶; - NH-R¹⁷; -NR¹⁸R¹⁹; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
with the proviso that at least one of the substituents R², R³, R⁴ and R⁵ represents a - N(R¹⁵)-S(=O)₂-R¹⁶ moiety;
R⁶, R⁹ and R¹¹, independent from one another, each represent a -NR²⁰R²¹ radical
or
a (hetero)cycloaliphatic radical selected from the group consisting of 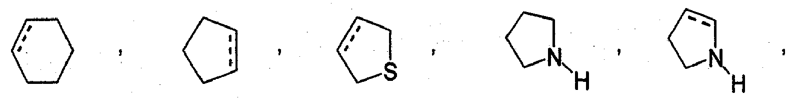 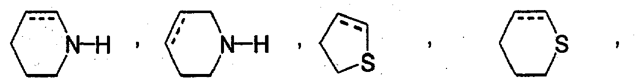 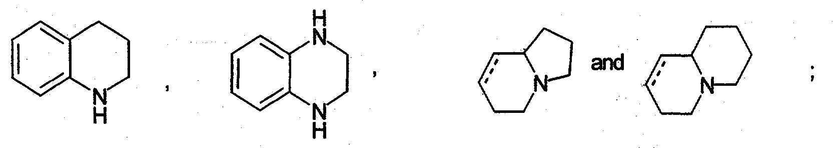 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, - O-CH₃, -O-C₂H₅, -S-CH₃, -S-C₂H₅, -C(=O)-OH, -C(=O)-O-CH₃, F, Cl, Br, I, -CN, - OCF₃, -SCF₃, -OH, -SH, -NH₂, -NH-CH₃, -NH-C₂H₅, -N(CH₃)₂, -N(C₂H₅)₂, -NO₂, - CHO, -CF₂H and -CFH₂ in any position including the -NH groups and is not bonded via a nitrogen atom and, if present, the dotted line represents an optional chemical bond;
R⁸ represents -C(=O)-R²²; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R¹⁰ represents a hydrogen atom; an alkyl radical selected from the group consisting of methyl, ethyl, n-propyl, isopropyl, n-butyl, sec-butyl, isobutyl and tert-butyl or a - S(=O)₂-R²³ moiety;
R¹², R¹³, R¹⁴, R¹⁷, R¹⁸ and R¹⁹, independent from one another, each represent an alkyl radical selected from the group consisting of methyl, ethyl, n-propyl, isopropyl, n-butyl, sec-butyl, isobutyl and tert-butyl; a (hetero)cycloaliptiatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, - O-C₂H₅, -S-CH₃, -C(=O)-OH, -C(=O)-O-CH₃, - F, Cl, Br, I, -CN, -OCF₃, -SCF₃, -OH, - SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
R¹⁵ represents a hydrogen atom; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or a -S(=O)₂-R²⁴ moiety;
R¹⁶ and R²⁴, independent from one another, each represent an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, - OCF₃, -SCF₃, -OH, -SH, -NH₂, -NH-CH₃, -NH-C₂H₅, -N(CH₃)₂, -N(C₂H₅)₂, -NO₂ phenyl, phenoxy and benzyl;
R²⁰ and R²¹, independent from one another, each represent a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, - NH2, -SH, -O-CH₃, -O-C₂H₅, -NO₂, -CN and -S-CH₃
or
R²⁰ and R²¹ together with the bridging nitrogen atom form a moiety selected from the group consisting of 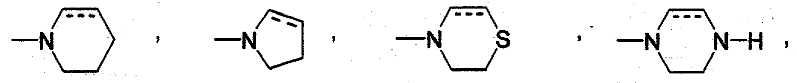 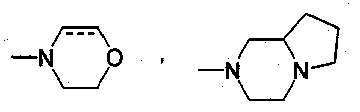 and 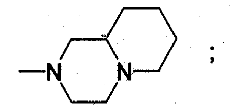 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, - NH-C₂H₅, -N(CH₃)₂ and -N(C₂H₅)₂ in any position including the -NH groups; and, if present, the dotted line represents an optional chemical bond;
R²² represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S- CH₃
and
R²³ represents an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo(=S), methyl, ethyl, -O-CH₃, -O-C₂H₅, -S-CH₃, -C(=O)-OH, -C(=O)-O-CH₃, - F, Cl, Br, I, -CN, - OCF₃, -SCF₃, -OH, -SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl), pyrrolyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or3}- group and which may be substituted with 1, 2 or 3 substituent(s) independently selected from the group consisting of -CF₃, methyl, ethyl, -O-CH₃, -O-C₂H₅, -O-CH₂-CH₂-CH₃, -S-CH₃, -S-C₂H₅, -C(=O)-OH, -C(=O)-O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also preferred are compounds of general formula I, wherein one of the substituents R², R³, R⁴ and R⁵ represents a -N(R¹⁵)-S(=O)₂-R¹⁶ moiety;
and X, Y, Z, n and R¹ to R²⁴ have any of the above defined meanings, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are compounds of general formula I, wherein one of the substituents R², R³, R⁴ and R⁵ represents a -N(R¹⁵)-S(=O)₂-R¹⁶ moiety and the other substituents R², R³, R⁴ and R⁵ each represent a hydrogen atom;
and X, Y, Z, n, R¹ and R⁶ to R²⁴ have any of the above defined meanings, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are compounds of general formula I, wherein R³ or R⁴ represents a - N(R¹⁵)-S(=O)₂-R¹⁶ moiety and the other substituents R², R³, R⁴ and R⁵ each represent a hydrogen atom;
and X, Y, Z, n, R¹ and R⁶ to R²⁴ have any of the above defined meanings, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore a substituted 1-H-indazolyl sulfonamide compound of general formula la is preferred, 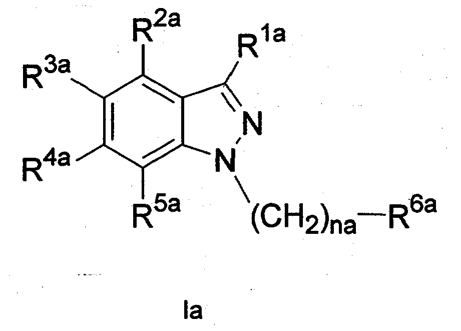 wherein
na is 0, 1 or 2;
R^{1a} represents a hydrogen atom; NO₂; -NH₂; -SH; -OH; -CN; F; Cl, Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, - NH₂, -SH, -O-CH₃, -O-C₂H₅, -NO₂, -CN and -S-CH₃;
R^{2a}, R^{3a}, R^{4a} and R^{5a}, independent from one another, each represent a hydrogen atom; -NO₂; -NH₂; -SH; -OH; -CN; -C(=O)-H; -C(=O)-R^{12a}; -OR^{13a}; -SR^{14a}; -N(R^{15a})-S(=O)₂-R^{16a}; -NH-R^{17a}; -NR^{18a}R^{19a}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, - NO₂, -CN and -S-CH₃;
with the proviso that at least one of the substituents R^{2a}, R^{3a}, R^{4a} and R^{5a} represents a -N(R^{15a})-S(=O)₂-R^{16a} moiety;
R^{6a} represents a -NR^{20a}R^{21a} radical
or
a (hetero)cycloaliphatic radical selected from the group consisting of 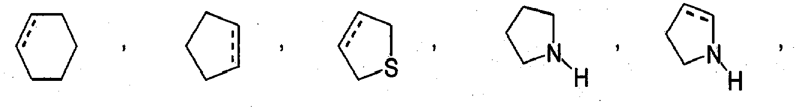 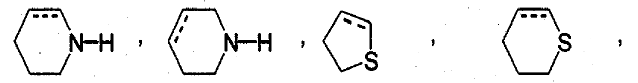 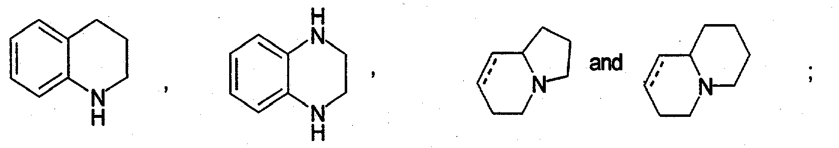 whereby each of these afore mentioned cyclic moieties may be substituted with 1, 2 or 3 substituent(s) independently selected from the group consisting of oxo(=O), thioxo (=S), methyl, ethyl, n-propyl, isopropyl, n-butyl, sec-butyl, isobutyl, tert-butyl, - O-CH₃, -O-C₂H₅, -S-CH₃, -S-C₂H₅, -C(=O)-OH, -C(=O)-O-CH₃, F, Cl, Br, I, -CN, - OCF₃, -SCF₃, -OH, -SH, -NH₂, -NH-CH₃, -NH-C₂H₅, --N(CH₃)₂, -N(C₂H₅)₂, -NO₂, - CHO, -CF₂H and -CFH₂ in any position including the -NH groups and is not bonded via a nitrogen atom and, if present, the dotted line represents an optional chemical bond;
R^{12a}, R^{13a}, R^{14a}, R^{17a}, R^{18a} and R^{19a}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, - O-C₂H₅, -S-CH₃, -C(=O)-OH, -C(=O)-O-CH₃, - F, Cl, Br, I, -CN, -OCF₃, -SCF₃, -OH,-SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
R^{15a} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R^{16a} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
and
R^{20a} and R^{21a}, independent from one another, each represent a hydrogen atom; or an alkyl radical selected from the group consisting of methyl, ethyl, n-propyl, isopropyl, n-butyl, sec-butyl, isobutyl and tert-butyl
or
R^{20a} and R^{21a} together with the bridging nitrogen atom form a moiety selected from the group consisting of 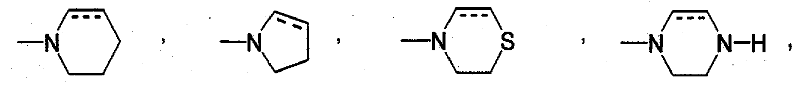 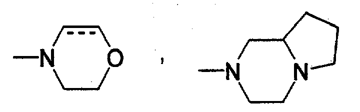 and 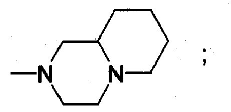 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, - NH-C₂H₅, -N(CH₃)₂ and -N(C₂H₅)₂ in any position including the -NH groups; and, if present, the dotted line represents an optional chemical bond;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Moreover such substituted 1-H-indazolyl sulfonamide compounds of general formula la are preferred, wherein
na is 0, 1 or 2;
R^{1a} represents a hydrogen atom or an alkyl radical selected from the group consisting of methyl, ethyl, n-propyl, isopropyl, n-butyl, sec-butyl, isobutyl and tert-butyl;
R^{2a}, R^{3a}, R^{4a} and R^{5a}, independent from one another, each represent a hydrogen atom; -NO₂; -NH₂; -SH; -OH; -CN; -N(R^{15a})-S(=O)₂-R^{16a}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2a}, R^{3a}, R^{4a} and R^{5a} represents a -N(R^{15a})-S(=O)₂-R^{16a} moiety;
R^{6a} represents a -NR^{20a}R^{21a} radical;
R^{15a} represents a hydrogen atom or an alkyl radical selected from the group consisting of methyl, ethyl, n-propyl, isopropyl, n-butyl, sec-butyl, isobutyl and tert-butyl
R^{16a} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl
and
R^{20a} and R^{21a}, independent from one another, each represent an alkyl radical selected from the group consisting of methyl, ethyl, n-propyl, isopropyl, n-butyl, sec-butyl, isobutyl and tert-butyl.
Moreover such substituted 1-H-indazolyl sulfonamide compounds of general formula la are preferred, wherein
na is 2;
R^{1a} represents a hydrogen atom or an alkyl radical selected from the group consisting of methyl, ethyl, n-propyl, isopropyl, n-butyl, sec-butyl, isobutyl and tert-butyl;
R^{2a}, R^{3a} and R^{5a}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
R^{4a} represents a -N(R^{15a})-S(=O)₂-R^{16a} moiety;
R^{6a} represents a -NR^{20a}R^{21a} radical;
R^{15a} represents a hydrogen atom;
R^{16a} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, F, Cl, Br, I, phenyl and phenoxy
and
R^{20a} and R^{21a} each represent a methyl radical.

Particularly, preferred are such substituted 1-H-indazolyl sulfonamide compounds of general formula la selected from the group consisting of
[1] N-(1-(2-(Dimethylamino)ethyl)-1H-indazol-6-yl)napthalene-2-sulfonamide and
[2] 5-Chloro-N-(1-(2-(dimethylamino)ethyl)-1H-indazol-6-yl)-3-methylbenzo[b]thiophene-2-sulfonamide;
   optionally in form of a physiologically acceptable salt thereof, or a corresponding solvate thereof.
   Furthermore a substituted 1-H-indazolyl sulfonamide compound of general formula Ib is preferred, 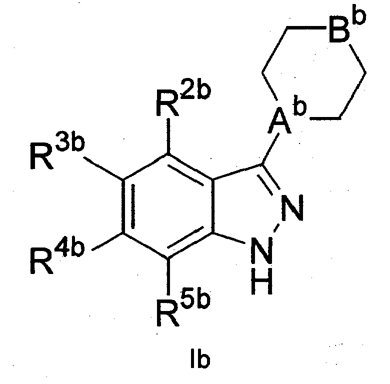 wherein
   A^{b} represents CH and B^{b} represents NR^{8b}
   or
   A^{b} represents N and B^{b} represents NR^{8b}
   or
   A^{b} represents N and B^{b} represents 0
   or
   A^{b} represents N and B^{b} represents S;
   R^{2b}, R^{3b}, R^{4b} and R^{5b}, independent from one another, each represent a hydrogen atom; -NO₂; -NH₂; -SH; -OH; -CN; -C(=O)-H; -C(=O)-R^{12b}; -OR^{13b}; -SR^{14b}; -N(R^{15b})-S(=O)₂-R^{16b}; -NH-R^{17b}; -NR^{18b}R^{19b}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, - NO₂, -CN and -S-CH₃;
   with the proviso that at least one of the substituents R^{2b}, R^{3b}, R^{4b} and R^{5b} represents a -N(R^{15b})-S(=O)₂-R^{16b} moiety;
   R^{8b} represents -C(=O)-R^{22b}; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
   R^{12b}, R^{13b}, R^{14b}, R^{17b}, R^{18b} and R^{19b}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, - O-C₂H₅, -S-CH₃, -C(=O)-OH, -C(=O)-O-CH₃, - F, Cl, Br, I, -CN, -OCF₃, -SCF₃, -OH, - SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
   R^{15b} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
   R^{16b} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which maybe substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
   and
   R^{22b} represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
   optionally in form of a physiologically acceptable salt thereof, or a corresponding solvate thereof.
   Moreover, such a substituted 1 H-indazolyl sulfonamidecompound of general formula Ib is preferred, wherein
   A^{b} represents CH and B^{b} represents NR^{8b}
   or
   A^{b} represents N and B^{b} represents NR^{8b}
   or
   A^{b} represents N and B^{b} represents O
   or
   A^{b} represents N and B^{b} represents S;
   R^{2b}, R^{3b}, R^{4b} and R^{5b}, independent from one another, each represent a hydrogen atom; -NO₂; -NH₂; -SH; -OH; -CN; -N(R^{15b})-S(=O)₂-R^{16b}; F; Cl; Br; I; or an alkyl radical selected from the group consisting of methyl, ethyl, n-propyl, isopropyl, n-butyl, sec-butyl, isobutyl and tert-butyl;
   with the proviso that at least one of the substituents R^{2b}, R^{3b}, R^{4b} and R^{5b} represents a -N(R^{15b})-S(=O)₂-R^{16b} Moiety;
   R^{8b} represents an alkyl radical selected from the group consisting of methyl, ethyl, n-propyl, isopropyl, n-butyl, sec-butyl, isobutyl and tert-butyl;
   R^{15b} represents a hydrogen atom or an alkyl radical selected from the group consisting of methyl, ethyl, n-propyl, isopropyl, n-butyl, sec-butyl, isobutyl and tert-butyl
   and
   R^{16b} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
   Moreover, such a substituted 1 H-indazolyl sulfonamide compound of general formula Ib is preferred, wherein
   A^{b} represents CH and B^{b} represents NR^{8b}
   R^{2b}, R^{4b} and R^{5b}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
   R^{3b} represents a -N(R^{15b})-S(=O)₂-R^{16b} moiety;
   R^{8b} represents a methyl radical;
   R^{15b} represents a hydrogen atom
   and
   R^{16b} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, tert-butyl, F, Cl, Br, I, phenyl and phenoxy.
   Particularly, preferred are such substituted 1-H-indazolyl sulfonamide compounds of general formula Ib selected from the group consisting of
[3] Naphthalene-2-sulfonic acid [3-(1-methyl-piperidin-4-yl)-1H-indazol-5-yl]-amide,
[4] 5-Chloro-3-methyl-benzo[b]thiophene-2-sulfonic acid [3-(1-methyl-piperidin-4-yl)-1H-indazol-5-yl]-amide,
[5] Naphthalene-1-sulfonic acid [3-(1-methyl-piperidin-4-yl)-1H-indazol-5-yl]-amide,
[6] 4-Phenylbenzene-4-sulfonic acid [3-(1-methyl-piperidin-4-yl)-1H-indazol-5-yl]-amide,
[7] N-[3-(1-Methyl-piperidin-4-yl)-1H-indazol-5-yl]-4-phenoxy-benzenesulfonamide
   and
[8] N-[3-(1-Methyl-piperidin-4-yl)-1H-indazol-5-yl]-benzenesulfonamide;
   optionally in form of a physiologically acceptable salt thereof, or a corresponding solvate thereof.
   Furthermore, such a substituted 1-H-indazolyl sulfonamide compound of general formula Ic is preferred, 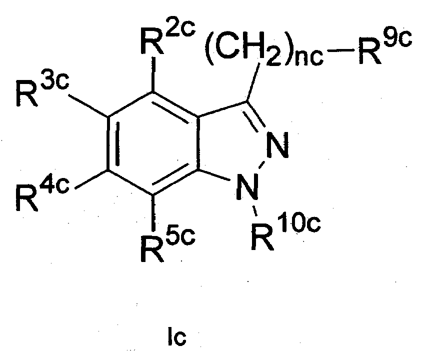 wherein
   nc is 0,1 or 2;
   R^{2c}, R^{3c}, R^{4c} and R^{5c}, independent from one another, each represent a hydrogen atom; -NO₂; -NH₂; -SH; -OH; -CN; -C(=O)-H; -C(=O)-R^{12c}; -OR^{13c}; -SR^{14c}; -N(R^{15c})-S(=O)₂-R^{16c}; -NH-R^{17c}; -NR^{18c}R^{19c}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, - NO₂, -CN and -S-CH₃;
   with the proviso that at least one of the substituents R^{2c}, R^{3c}, R^{4c} and R^{5c} represents a -N(R^{15c})-S(=O)₂-R^{16c} moiety;
   R^{9c} represents a -NR^{20c}R^{21c} radical
   or
   a (hetero)cycloaliphatic radical selected from the group consisting of 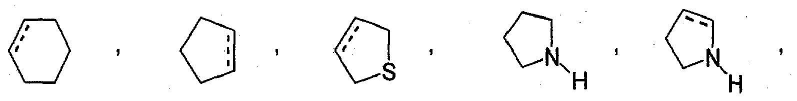 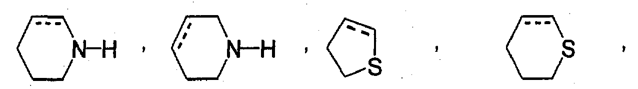 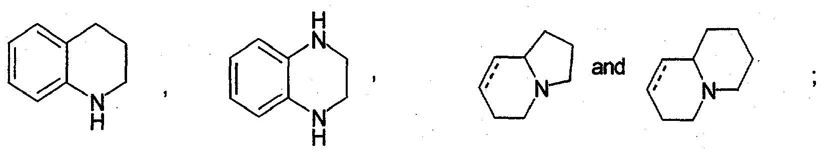 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, - O-CH₃, -O-C₂H₅, -S-CH₃, -S-C₂H₅, -C(=O)-OH, -C(=O)-O-CH₃, F, Cl, Br, I, -CN, - OCF₃, -SCF₃, -OH, -SH, -NH₂, -NH-CH₃, -NH-C₂H₅, --N(CH₃)₂, -N(C₂H₅)₂, -NO₂, - CHO, -CF₂H and -CFH₂ in any position including the -NH groups and is not bonded via a nitrogen atom and, if present, the dotted line represents an optional chemical bond;
   R^{10c} represents a hydrogen atom; an alkyl radical selected from the group consisting of methyl, ethyl, n-propyl, isopropyl, n-butyl, sec-butyl, isobutyl and tert-butyl or a - S(=O)₂-R^{23c} moiety;
   R^{12c}, R^{13c}, R^{14c}, R^{17c}, R^{18c} and R^{19c}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, - O-C₂H₅, -S-CH₃, -C(=O)-OH, -C(=O)-O-CH₃, - F, Cl, Br, I, -CN, -OCF₃, -SCF₃, -OH, - SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
   R^{20c} and R^{21c}, independent from one another, each represent a hydrogen atom; or an alkyl radical selected from the group consisting of methyl, ethyl, n-propyl, isopropyl, n-butyl, sec-butyl, isobutyl and tert-butyl;
   or
   R^{20c} and R^{21c} together with the bridging nitrogen atom form a moiety selected from the group consisting of 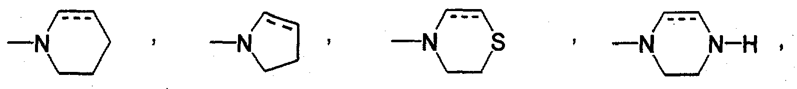 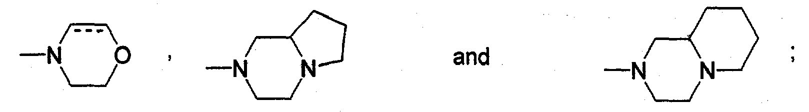 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, - NH-C₂H₅, -N(CH₃)₂ and -N(C₂H₅)₂ in any position including the -NH groups; and, if present, the dotted line represents an optional chemical bond
   and
   R^{23c} represents an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo {=S), methyl, ethyl, -O-CH₃, -O-C₂H₅, -S-CH₃, -C(=O)-OH, -C(=O)-O-CH₃, - F, Cl, Br, I, -CN, - OCF₃, -SCF₃, -OH, -SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl), pyrrolyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, -O-CH₃, -0-C₂H₅, -O-CH₂-CH₂-CH₃, -S-CH₃, -S-C₂H₅, -C(=O)-OH, -C(=O)=O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
   optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.
   Moreover, such a substituted 1 H-indazolyl sulfonamide compound of general formula Ic is preferred, wherein
   nc is 0, 1 or 2;
   R^{2c}, R^{3c}, R^{4c} and R^{5c}, independent from one another, each represent a hydrogen atom; -NO₂; -NH₂; -SH; -OH; -CN; -N(R^{15c})-S(=O)₂-R^{16c}; F; Cl; Br; I; or an alkyl radical selected from the group consisting of methyl, ethyl, n-propyl, isopropyl, n-butyl, sec-butyl, isobutyl and tert-butyl;
   with the proviso that at least one of the substituents R^{2c}, R^{3c}, R^{4c} and R^{5c} represents a -N(R^{15c})-S(=O)₂-R^{16c} moiety;
   R^{9c} represents a -NR^{20c}R^{21c} radical;
   R^{10C} represents a hydrogen atom; an alkyl radical selected from the group consisting of methyl, ethyl, n-propyl, isopropyl, n-butyl, sec-butyl, isobutyl and tert-butyl or a - S(=O)₂-R^{23c} moiety;
   R^{15c} represents a hydrogen atom or an alkyl radical selected from the group consisting of methyl, ethyl, n-propyl, isopropyl, n-butyl, sec-butyl, isobutyl and tert-butyl
   R^{16c} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
   R^{20c} and R^{21c}, independent from one another, each represent an alkyl radical selected from the group consisting of methyl, ethyl, n-propyl, isopropyl, n-butyl, sec-butyl, isobutyl and tert-butyl;
   or
   R^{20c} and R^{21c} together with the bridging nitrogen atom form an unsubstituted moiety selected from the group consisting of 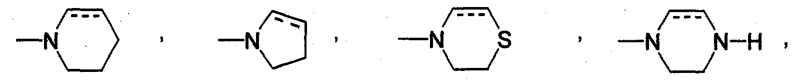 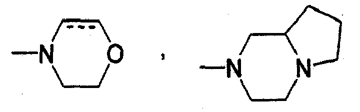 and 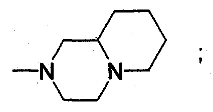 wherein, if present, the dotted line represents an optional chemical bond;
   and
   R^{23c} represents an alkyl radical selected from the group consisting of methyl, ethyl, n-propyl, isopropyl, n-butyl, sec-butyl, isobutyl and tert-butyl
   or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
   Moreover, such a substituted 1H-indazolyl sulfonamide compound of general formula Ic is preferred, wherein
   nc is 0, 1 or 2;
   R^{2c}, R^{3c}, R^{4c} and R^{5c}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
   with the proviso that either R^{3c} or R^{4c} represents a -N(R^{15c})-S(=O)₂-R^{16c} moiety;
   R^{9c} represents a -NR^{20c}R^{21c} radical;
   R^{10c} represents a hydrogen atom; an alkyl radical selected from the group consisting of methyl, ethyl, n-propyl, isopropyl, n-butyl, sec-butyl, isobutyl and tert-butyl or a - S(=O)₂-R^{23c} moiety;
   R^{15c} represents a hydrogen atom;
   R^{16c} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, tert-butyl, F, Cl, Br and I;
   R^{20c} and R^{21c}, independent from one another, each represent an alkyl radical selected from the group consisting of methyl, ethyl, n-propyl, isopropyl, n-butyl, sec-butyl, isobutyl and tert-butyl
   and
   R^{23c} represents an alkyl radical selected from the group consisting of methyl, ethyl, n-propyl, isopropyl, n-butyl, sec-butyl, isobutyl and tert-butyl
   or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
   In another aspect of the present invention, a substituted 2,3-dihydro-indolyl compound of general formula Id is preferred, 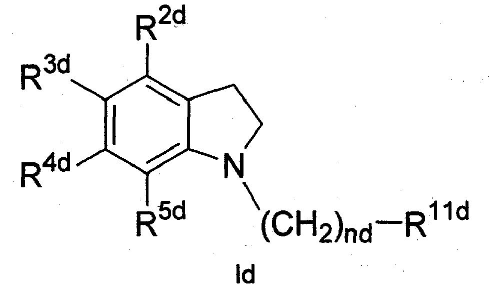 wherein
   nd is 0, 1 or 2;
   R^{2d}, R^{3d}, R^{4d} and R^{5d}, independent from one another, each represent a hydrogen atom; -NO₂; -NH₂; -SH; -OH; -CN; -C(=O)-H; -C(=O)-R^{12d}; -OR^{13d}; -SR^{14d}; -N(R^{15d})-S(=O)₂-R^{16d}; -NH-R^{17d}; -NR^{18d}R^{19d}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, - NO₂, -CN and -S-CH₃;
   with the proviso that at least one of the substituents R^{2d}, R^{3d}, R^{4d} and R^{5d} represents a -N(R^{15d})-S(=O)₂-R^{16d} moiety;
   R^{11d} represents a -NR^{20d}R^{21d} radical
   or
   a (hetero)cycloaliphatic radical selected from the group consisting of 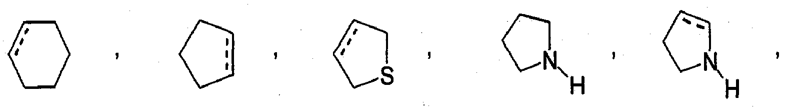 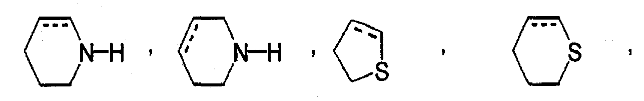 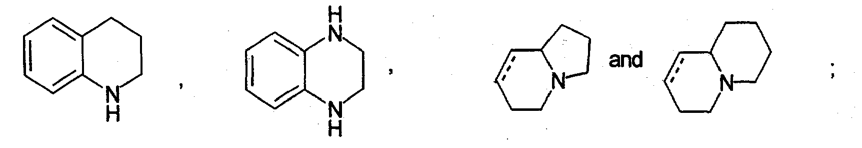 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, - O-CH₃, -O-C₂H₅, -S-CH₃, -S-C₂H₅, -C(=O)-OH, -C(=O)-O-CH₃, F, Cl, Br, I, -CN, - OCF₃, -SCF₃, -OH, -SH, -NH₂, -NH-CH₃, -NH=C₂H₅, --N(CH₃)₂, -N(C₂H₅)₂, -NO₂, - CHO, -CF₂H and -CFH₂ in any position including the -NH groups and is not bonded via a nitrogen atom and, if present, the dotted line represents an optional chemical bond;
   R^{12d}, R^{13d}, R^{14d}, R^{17d}, R^{18d} and R^{19d}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or} 3- group and which may be substituted with 1, 2 or 3 substituent(s) independently selected from the group consisting of oxo (=O), thioxo (=S), methyl, ethyl, -O-CH₃, - O-C₂H₅, -S-CH₃, -C(=O)-OH, -C(=O)-O-CH₃, - F, Cl, Br, I, -CN, -OCF₃, -SCF₃, -OH, - SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, -ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
   R^{15d} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
   R^{16d} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
   and
   R^{20d} and R^{21d}, independent from one another, each represent a hydrogen atom; or an alkyl radical selected from the group consisting of methyl, ethyl, n-propyl, isopropyl, n-butyl, sec-butyl, isobutyl and tert-butyl;
   or
   R^{20d} and R^{21d} together with the bridging nitrogen atom form a moiety selected from the group consisting of 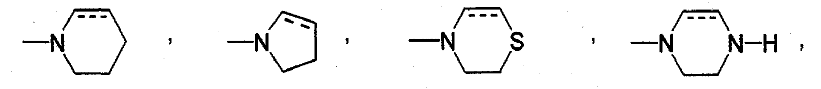 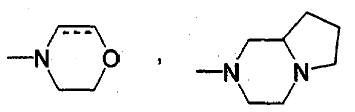 and 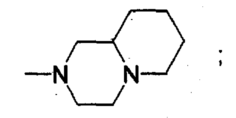 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, - NH-C₂H₅, -N(CH₃)₂ and -N(C₂H₅)₂ in any position including the -NH groups; and, if present, the dotted line represents an optional chemical bond;
   optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.
   Moreover, such a substituted 2,3-dihydro-indolyl compound of general formula Id is preferred, wherein
   nd is 0, 1 or 2;
   R^{2d}, R^{3d}, R^{4d} and R^{5d}, independent from one another, each represent a hydrogen atom; -NO₂; -NH₂; -SH; -OH; -CN; -N(R^{15d})-S(=O)₂-R^{16d}; F; Cl; Br; I; or an alkyl radical selected from the group consisting of methyl, ethyl, n-propyl, isopropyl, n-butyl, sec-butyl, isobutyl and tert-butyl;
   with the proviso that at least one of the substituents R^{2d}, R^{3d}, R^{4d} and R^{5d} represents a -N(R^{15d})-S(=O)₂-R^{16d} moiety;
   R^{11d} represents a -NR^{20d}R^{21d} radical;
   R^{15d} represents a hydrogen atom or an alkyl radical selected from the group consisting of methyl, ethyl, n-propyl, isopropyl, n-butyl, sec-butyl, isobutyl and tert-butyl;
   R^{16d} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl
   and
   R^{20d} and R^{21d}, independent from one another, each represent an alkyl radical selected from the group consisting of methyl, ethyl, n-propyl, isopropyl, n-butyl, sec-butyl, isobutyl and tert-butyl.
   Moreover, such a substituted 2,3-dihydro-indolyl compound of general formula Id is preferred, wherein
   nd is 2;
   R^{2d}, R^{3d} and R^{5d}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
   R^{4d} represents a -N(R^{15d})-S(=O)₂-R^{16d} moiety;
   R^{11d} represents a -NR^{20d}R^{21d} radical;
   R^{15d} represents a hydrogen atom;
   R^{16d} represents an imidazo[2,1-b]thiazolyl radical, which may be substituted with 1, 2 or 3 substituent(s) independently selected from the group consisting of F, Cl, Br and I and
   R^{20d} and R^{21d} each represent a methyl radical.
   Particularly preferred is the following compound of general formula Id
[9] N-[1-(2-Dimethylamino)ethyl)-2,3-dihydro-1H-indol-6-yl]-6-chloro-imidazo[2,1-b]thiazol-5-sulfonamide;
   optionally in form of a physiologically acceptable salt thereof, or a corresponding solvate thereof.

Particularly preferred is a compound of general formula I, wherein
X-Y from left to right represents CR¹=N and Z is N[(CH₂)ₙR⁶]
or
X-Y from left to right represents CR⁷=N, Z is NH, R⁷ represents the following moiety 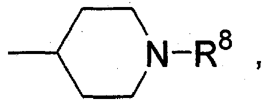 or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1 or 2;
R¹ represents a hydrogen atom or an alkyl radical selected from the group consisting of methyl, ethyl, n-propyl, isopropyl, n-butyl, sec-butyl, isobutyl and tert-butyl;
R², R³, R⁴ and R⁵, independent from one another, each represent a hydrogen atom; F; Cl; Br; I; an -N(R¹⁵)-S(=O)₂-R¹⁶- moiety, or an alkyl radical selected from the group consisting of methyl, ethyl, n-propyl, isopropyl, n-butyl, sec-butyl, isobutyl and tert-butyl;
with the proviso that either R³ or R⁴ represents a -N(R¹⁵)-S(=O)₂-R¹⁶ moiety;
R⁶ represents a -NR²⁰R²¹ radical;
R⁸ represents an alkyl radical selected from the group consisting of methyl, ethyl, n-propyl, isopropyl, n-butyl, sec-butyl, isobutyl and tert-butyl;
R⁹ represents a -NR²⁰R²¹ radical;
R¹⁰ represents a hydrogen atom; an alkyl radical selected from the group consisting of methyl, ethyl, n-propyl, isopropyl, n-butyl, sec-butyl, isobutyl and tert-butyl or a - S(=O)₂-R²³ moiety;
R¹¹ represents a -NR²⁰R²¹ radical;
R¹⁵ represents a hydrogen atom;
R¹⁶ represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, F, Cl, Br and I;
R²⁰ and R²¹, independent from one another, each represent an alkyl radical selected from the group consisting of methyl, ethyl, n-propyl, isopropyl, n-butyl, sec-butyl, isobutyl and tert-butyl
and
R²³ represents an alkyl radical selected from the group consisting of methyl, ethyl, n-propyl, isopropyl, n-butyl, sec-butyl, isobutyl and tert-butyl
or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. they may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, 0 and S. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

An alkenyl radical according to the present invention comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, iso-butyl, sec-butyl, tert-butyl, n-pentyl, sec-pentyl, neo-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl), -N(C₁₋₅-alkyl)₂ and phenyl, whereby in each occurence C₁₋₅-alkyl may be linear or branched and the phenyl radical is preferably unsubstituted. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃₋,-(CH₂)₄-,-(CH₂)₅ and -(CH₂)₆-, suitable alkenylene groups include -CH=CH-, -CH₂₋CH=CH- and -CH=CH-CH₂- and suitable alkinylene groups include -C≡C-, -CH₂₋C≡C- and -C≡C-CH₂-.

If the alkylene, alkenylene or alkinylene group contains one or more, preferably 1, 2 or 3, more preferably 1, heteroatom(s) as chain member(s), unless defined otherwise, each of these heteroatom(s) may preferably be selected from the group consisting of N, 0 and S. Suitable alkylene groups which contain one or more heteroatom(s) include -CH₂-O-CH₂- and -CH₂-CH₂-O.

Another aspect of the present invention relates to a process for the preparation of a substituted indazolyl sulfonamide or 2,3-dihydro-indolyl sulfonamide compound of general formula I, Ia, Ib, Ic or Id given above, wherein at least one compound of general formula II, 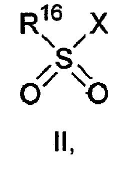 wherein R¹⁶ has the meaning given above and X represents a leaving group, preferably a halogen atom, more preferably a chlorine atom, is reacted with at least one compound of general formula III, 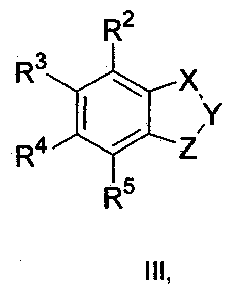 wherein X, Y, Z and R² to R⁵ have the meaning given above with the proviso that at least one of the substituents R², R³, R⁴ and R⁵, represents a -NH₂ group, in a suitable reaction medium, preferably in the presence of at least one base, to yield a compound of general formula I, wherein X, Y, Z and R² to R⁵ have the meaning given above with the proviso that at least one of the substituents R², R³, R⁴ and R⁵ represents a -N(H)-S(=O)₂-R¹⁶ group and R¹⁶ has the meaning given above, which is optionally purified and/or isolated,
and optionally said compound of general formula I, wherein X, Y, Z and R² to R⁵ have the meaning given above with the proviso that at least one of the substituents R², R³, R⁴ and R⁵ represents a -N(H)-S(=O)₂-R¹⁶ group and R¹⁶ has the meaning given above, is reacted with at least one compound of general formula R¹⁵-X, wherein R¹⁵ has the meaning given above and X represents a halogen atom, preferably a chlorine atom, in a suitable reaction medium, in the presence of at least one base, preferably at least one base selected from the group consisting of metal hydroxides, metal carbonates, metal alcoxides, preferably sodium methoxide or potassium tert-butoxid, metal hydrides and organometallic compounds, preferably n-butyllithium and tert-butyllithium,
or with at least one compound of general formula X-S(=O)₂-R²⁴, wherein R²⁴ has the meaning given above and X represents a leaving group, preferably a halogen atom, more preferably a chlorine atom, in a suitable reaction medium, preferably in the presence of at least one base,
to yield a compound of general formula I, wherein X, Y, Z and R² to R⁵ have the meaning given above with the proviso that at least one of the substituents R², R³, R⁴ and R⁵ represents a -N(R¹⁵)-S(=O)₂-R¹⁶ group and R¹⁵ and R¹⁶ have the meaning given above, which is optionally purified and/or isolated.

Suitable reaction media for the reaction between compounds of general formulas II and III include organic solvents, such as dialkyl ether, preferably diethyl ether, or a cyclic ether, preferably tetrahydrofuran or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The reaction between compounds of general formulas II and III is preferably carried out in the presence of at least one suitable base, for example, an inorganic base such as a hydroxide or a carbonate of an alkali metal and/or an organic base, preferably triethylamine or pyridine.

The reaction between compounds of general formulas II and III is preferably carried out at a temperature between -10 °C and ambient temperature, i.e. approximately 25 °C and the reaction time is preferably between 5 minutes and 34 hours.

Suitable reaction media for the reaction between compounds of general formula I, wherein X, Y, Z and R² to R⁵ have the meaning given above with the proviso that at least one of the substituents R², R³, R⁴ and R⁵ represents a -N(H)-S(=O)₂-R¹⁶ group and R¹⁶ has the meaning given above and compounds of general formula R¹⁵-X are dialkyl ether, preferably diethyl ether, or a cyclic ether, preferably tetrahydrofuran or dioxane, a hydrocarbon, preferably toluene, an alcohol, preferably methanol or ethanol, a dipolar aprotic solvent, preferably acetonitrile, pyridine or dimethylformamide, or any other suitable reaction medium. Of course, mixtures of at least two classes of solvents or of at least two solvents of one class may also be used.

The afore mentioned reaction is preferably carried out at a temperature between - 10 °C and ambient temperature, i.e. approximately 25 °C and the reaction time is preferably 1 and 24 hours.

Suitable reaction media for the reaction between compounds of general formula I, wherein X, Y, Z and R² to R⁵ have the meaning given above with the proviso that at least one of the substituents R², R³, R⁴ and R⁵ represents a -N(H)-S(=O)₂-R¹⁶ group and R¹⁶ has the meaning given above, and compounds of general formula X-S(=O)₂₋R²⁴ include organic solvents, such as dialkyl ether, preferably diethyl ether, or a cyclic ether, preferably tetrahydrofuran or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The afore mentioned reaction is preferably carried out in the presence of at least one suitable base, for example, an inorganic base such as a hydroxide or a carbonate of an alkali metal and/or an organic base, preferably triethylamine or pyridine.

The afore mentioned reaction is preferably carried out at a temperature between - 10 °C and ambient temperature, i.e. approximately 25 °C and the reaction time is preferably between 5 minutes and 24 hours.

Those skilled in the art understand that the process described above-can also be applied to the synthesis of compounds of general formula la, Ib, Ic and Id given above.

The compounds of general formula I, Ia, Ib, Ic or Id given above may be purified and/or isolated according to methods well known to those skilled in the art. Preferably, the compounds of general formula I, la, Ib, Ic or Id may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formula II are commercially available or maybe prepared according to methods well known in the art, for example, analogous to the methods described in the bibliography of E.E. Gilbert, Synthesis, 1969, 1, 3. The respective part of the literature description cited above is hereby incorporated by reference and forms part of the disclosure.

The compounds of general formula III are commercially available or may also be prepared according to standard methods known in the prior art, for example by methods similar to those described in the literature: Savitskaya, N. V. et al. Synthesis of 5-amino-3-(b-aminoethyl)indazole. Zhurnal Obshchei Khimii (1961), 31 1924-1926; Zhang, Han-Cheng et al. Discovery and Optimization of a Novel Series of Thrombin Receptor (PAR-1) Antagonists: Potent, Selective Peptide Mimetics Based on indole and Indazole Templates. Journal of Medicinal Chemistry (2001), 44(7), 1021-1024; Ono, Shinichiro et al. Preparation of piperidine derivatives as muscarinic receptors stimulator for treatment of schizophrenia. WO 2004069828 Al; Wrzeciono, U. et al. Synthesis and antiinflammatory activity of some indazole derivatives. Part 36. Azoles. Pharmazie (1993), 48(8); 582-584.; Filla, S. A. et al. Preparation o 3-(1-methylpiperidin-4-yl)-1H-indoles and 3-(1-methylpiperidin-4-yl)4-aza-1H-indoles as 5-HT1F agonist. WO 2000/487; Dumas, J. et al. Preparation of bicyclic (hetero)aryl-and pyridine-containing diaryl ureas as Raf kinase and angiogenesis inhibitors useful in the treatment of cancer and other disorders. WO 200478748; Mueller, S. G. et al. Preparation of ethynylpyridines and related compounds as melanin-concentrating hormone receptor (MCH-1) antagonist for the treatment of metabolic disorders. WO 200439780; Maeno, K. Preparation of aminoalkylindazole derivatives as 5-HT2c receptor agonists. WO 98/30548; Zhao, E.-C. et al. Synthesis of dialkylaminoalkyl derivatives of indazole. Zhurnal Obshchei Khimii (1959), 29, 1012-1020. Ham, P. et al. Preparation of N-heteroaryl-4'-oxadiazolylbiphenylcarboxamides as 5HT1D antagonists. WO 9532967A1; Stenkamp, D. et al. Preparation of arylamides as melanin concentrating hormone (MCH) receptor antagonists. WO 200403974.

One of them consists of nitro group reduction of derivatives of general formula IV, 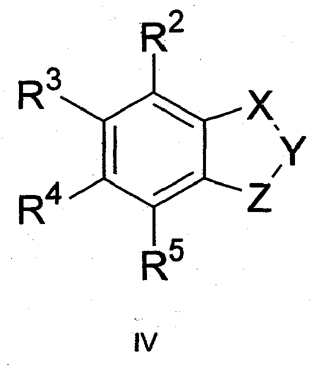 wherein R² to R⁵, X, Y and Z have the meaning given above; with the proviso that at least one substituent of the group consisting of R², R³, R⁴ and R⁵ represents -NO₂; or one of their suitably protected derivatives by methods known in the prior art, as for example: Wrzeciono, U. et al. Azoles. Part 10: Behavior of 2,4-dinitroindazole on aliphatic and cyclic amines. Pharmazie (1983), 38(2), 85-88; Wrzeciono, U. et al. Azoles. Part 3. Nitro derivatives of 3-chloroindazole and the effect of G-5, C-6, and N-nitro groups on the reactivity of the C-3 chlorine atom. Pharmazie (1978), 33(7), 419-424.; Wrzeciono, U. Azoles. Part 11: behavior of 2,7-dinitroindazole in relation to cyclic and aliphatic amines. Pharmazie (1984), 39(6), 389-391; Beauge, F. et al. 3-substituted 7-nitroindazoles, process for their preparation, and their therapeutic use, particularly for the treatment of alcohol dependence. FR 2832406 A1 20030523; Sarbach, R. Pharmacologically active indazole derivatives. FR 7631 19700126; Dumont, J. M. et al. Synthesis and anthelmintic activity of indazoles and nitroindazoles. European Journal of Medicinal Chemistry (1983), 18, 469-470; Wrzeciono, U. et al. Azoles. Part 8: N1-aminomethyl derivatives of 5-nitro- and 3-chloro- or 3-bromo-5-nitroindazole. Pharmazie (1980), 35, 746-748; Andersson, C.-M. A. et al. Preparation of heterocyclic pharmaceutical compositions as muscarinic agonists. WO 2001/83472; Ham, P. et al. Preparation of N-heteroaryl-4'-oxadiazolylbiphenylcarboxamides as 5HT1D antagonists. WO 9532967A1; Auvin, S. et al. Novel amidine derivatives, their preparation and application as inhibitors of NO synthase and lipid peroxidation, and pharmaceutical compositions containing them. WO 200017190; and, if necessary, the protective groups are removed in order to obtain the corresponding amine of general formula III, which may be purified and/or isolated by means of conventional methods known in the prior art. The respective parts of the literature descriptions cited above are hereby incorporated by reference and form part of the disclosure.

The compounds of general formula IV are commercially available or may also be prepared according to standard methods known in the prior art.

The compounds of general formula I, la, Ib, Ic or Id given above may be purified and/or isolated according to methods well known to those skilled in the art. Preferably, the compounds of general formula I, la, lb, lc or ld may be isolated by evaporating the reaction medium, addition of water and then adjusting the pH value to obtain the compound in form of a solid that can be isolated by filtration, or by extraction with a solvent that is not miscible with water such as chloroform and purified by chromatography or recrystallisation from a suitable solvent.

During some synthetic reactions described above or while preparing the compounds of general formulas I, la, Ib, Ic, Id, II, III or IV the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indazolyl sulfonamide or 2,3-dihydro-indolyl sulfonamide compounds of general formula I, la, Ib, Ic or Id are obtained in form of a mixture of stereoisomers, particularly enantiomers or diastereomers, said mixtures maybe separated by standard procedures known to those skilled in the art, e.g. chromatographic methods or crystallization with chiral reagents.
The substituted indazolyl sulfonamide or 2,3-dihydro-indolyl sulfonamide compounds of general formula I, la, Ib, lc or ld and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

The term "salt" is to be understood as meaning any form of the substituted indazolyl sulfonamide or 2,3-dihydro-indolyl sulfonamide compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted indazolyl sulfonamide or 2,3-dihydro-indolyl sulfonamide compounds of general formula I, Ia, Ib, Ic or Id in each case of corresponding stereoisomers may also be obtained by standard procedures known to those skilled in the art.

The term "solvate" according to this invention is to be understood as meaning any form of the substituted indazolyl sulfonamide or 2,3-dihydro-indolyl sulfonamide compounds in which they have attached to it via non-covalent binding another molecule (most likely a polar solvent) especially including hydrates and alcoholates, e.g. methanolate.

A further aspect of the present invention relates to a medicament comprising at least one substituted indazolyl sulfonamide or 2,3-dihydro-indolyl sulfonamide compound of general formula I, la, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.

Said medicament is particularly suitable for 5-HT₆-receptor regulation and therefore for the prophylaxis and/or treatment of a disorder or a disease that is least partially mediated via 5-HT₆-receptors.

Preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

More preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Furthermore, more preferably said medicament is suitable for improvement of cognition (cognitive enhancement) or cognitive memory (cognitive memory enhancement).

Furthermore, more preferably said medicament is suitable for the prophylaxis and/or treatment of drug addiction and/or withdrawal; for the prophylaxis and/or treatment of alcohol addiction and/or withdrawal or for the prophylaxis and/or treatment of nicotine addiction and/or withdrawal.

Most preferably, said medicament is suitable for the prophylaxis and/or treatment of obesity and/or disorders or diseases related thereto.

In another aspect the present invention relates to the use of at least one substituted indazolyl sulfonamide or 2,3-dihydro-indolyl sulfonamide compound of general formula I, Ia, Ib, Ic or Id given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

The use of at least one substituted indazolyl sulfonamide or 2,3-dihydro-indolyl sulfonamide compound of general formula I, Ia, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity is preferred.

The use of at least one substituted indazolyl sulfonamide or 2,3-dihydro-indolyl sulfonamide compound of general formula I, Ia, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder) is preferred.

The use of at least one substituted indazolyl sulfonamide or 2,3-dihydro-indolyl sulfonamide compound of general formula I, Ia, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement) is preferred.

The use of at least one substituted indazolyl sulfonamide or 2,3-dihydro-indolyl sulfonamide compound of general formula I, Ia, lb, Ic or ld given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine is preferred.

More preferred is the use of at least one substituted indazolyl sulfonamide or 2,3-dihydro-indolyl sulfonamide compound of general formula I, Ia, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

More preferred is the use of at least one substituted indazolyl sulfonamide or 2,3-dihydro-indolyl sulfonamide compound of general formula I, Ia, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the improvement of cognition (cognitive enhancement) and/or for the improvement of cognitive memory (cognitive memory enhancement).

More preferred is the use of at least one substituted indazolyl sulfonamide or 2,3-dihydro-indolyl sulfonamide compound of general formula I, la, Ib, Ic or Id given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.

Most preferred is the use of at least one substituted indazolyl sulfonamide or 2,3-dihydro-indolyl sulfonamide compound of general formula I, la, Ib, Ic or Id as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of obesity and/or disorders or diseases related thereto.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C. T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Ppess Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2,698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective indazolyl sulfonamide or 2,3-dihydro-indolyl sulfonamide compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more substituted indazolyl sulfonamide or 2,3-dihydro-indolyl sulfonamide compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000 mg, preferably 1 to 1500 mg, more preferably 1 to 1000 milligrams of active substance to be administered during one or several intakes per day.

In the following methods for determining the pharmacological activity of the substituted indazolyl sulfonamide or 2,3-dihydro-indolyl sulfonamide compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl_{2,} 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted indazolyl sulfonamide or 2,3-dihydro-indolyl sulfonamide compounds according to the present invention in fasted rats is then determined as follows:
The rats were fasted for 23 hours in their single homecages. After this period, the rats are orally or intraperitoneally dosed with a composition comprising a substituted indazolyl sulfonamide or 2,3-dihydro-indolyl sulfonamide compound or a corresponding composition (vehicle) without said substituted indazolyl sulfonamide or 2,3-dihydro-indolyl sulfonamide compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples:

### 1. N-(1-(2-(dimethylamino)ethyl)-1H-indazol-6-yl)napthalene-2-sulfonamide

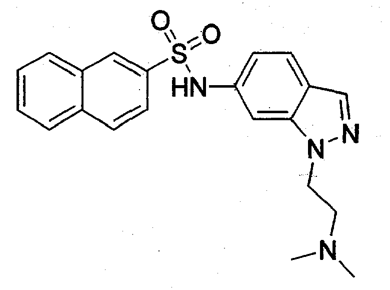

To a solution containing 204 mg (1.0 mmol) of 1-(2-dimethylamino)ethyl)-1H-indazol-6-yl-amine and 193 mg N-diisopropylethylamine in 5 ml dimethylformamide was added 250 mg (1.1 mmol) naphthalene sulfonyl chloride. The reaction mixture was stirred at ambient temperature for 8 hours. The solvent was evaporated and the residue was taken up in a saturated aqueous NaHCO₃ solution and extracted with chloroform. The combined organic phases were dried over Na₂SO₄ and concentrated in vacuo. The residue was purified by column chromatography to yield 200 mg (50 %) of the desired compound.

¹H-NMR (300 MHz, CDCl₃): δ 2.21 (s, 6H); 2.71 (m, 2H); 4.37(m, 2H); 6,77 (dd, 1H, J = 8.6 Hz, J' = 1.9 Hz); 7.30 (m, 1 H); 7.50-7.61 (m, 3H); 7.75 (m, 1 H); 7.82-789 (m, 4H); 8.38 (m, 1 H)

### 2. 5-chloro-N-(1-(2-(dimethylamino)ethyl)-1H-indazol-6-yl)-3-methylbenzo[b]thiophene-2-sulfonamide

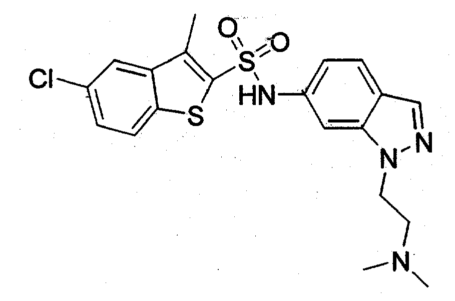

To a solution containing 204 mg (1.0 mmol) of 1-(2-dimethylamino)ethyl)-1H-indazol-6-yl-amine and 193 mg N-diisopropylethylamine in 5 ml dimethylformamide was added 309 mg (1.1 mmol) 5-chloro-3-methylbenzo[b]thiophene sulfonyl chloride. The reaction mixture was stirred at ambient temperature for 8 hours. The solvent was evaporated and the residue was taken up in a saturated aqueous NaHCO₃ solution and extracted with chloroform. The combined organic phases were dried over Na₂SO₄ and concentrated in vacuo. The residue was purified by column chromatography to yield 179 mg (40 %) of the desired compound.

¹H-NMR (300 MHz, DMSO-d₆): δ 2.04 (s, 6H); 2.46 (s, 3H); 2.57 (m, 2H); 4.32 (m, 2H); 6.88 (d, 1H, J = 8.6 Hz); 7.28 (s, 1 H); 7.52 (dd, 1H, J = 8.6 Hz, J' = 1.6 Hz); 7.59 (d, 1 H, J = 8.6 Hz); 7.91 (s, 1 H); 7.96 (d, 1 H, J = 1.5 Hz); 8.00 (d, 1 H, J = 8.6 Hz)

### 3. naphthalene-2-sulfonic acid [3-(1-methyl-piperidin-4-yl)-1H-indazol-5-yl]-amide

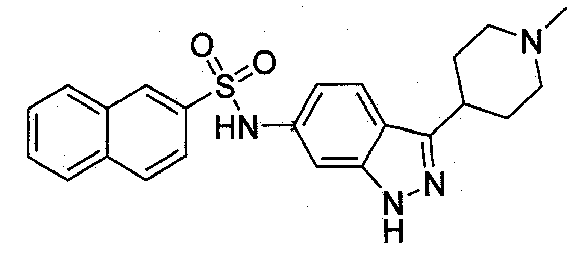

124.5 mg (0.55 mmol) Naphthalene-sulfonyl chloride was added to a solution containing 115 mg (0.5 mmol) of 3-(1-methyl-piperidin-4-yl)-1H-indazol-5-yl-amine and 150 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 115 mg (55 %) of the desired product.

¹H-NMR (300 MHz, DMSO-d₆): δ 1.61 (m, 4H); 1.89 (m, 2H); 2.16 (s, 3H); 2.63-2.76 (m, 3H); 7.02 (d, 1 H, J = 8.6 Hz); 7.21 (s, 1H); 7.29 (d, 1 H, J = 9.0 Hz); 7.55 - 7.62 (m, 1 H); 7.62 - 7.68 (m, 1 H); 7.69 - 7.75 (m, 1 H); 7.97(d, 1 H, J = 8.1 Hz); 7.99-8.07 (m, 2H); 8.26 (s, 1 H); 10.01 (b, 1 H); 12.56 (s, 1 H)

### 4. 5-chloro-3-methyl-benzo[b]thiophene-2-sulfonic acid [3-(1-methyl-piperidin-4-yl)-1H-indazol-5-yl]-amide

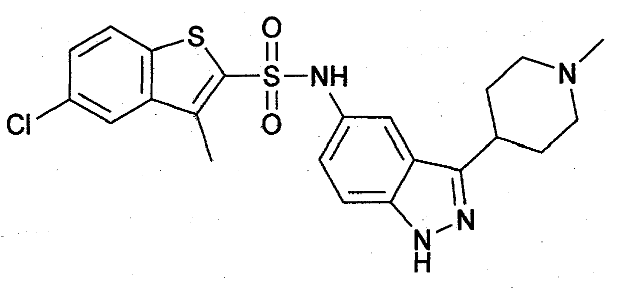

124 mg (0.44 mmol) 5-Chloro-3-methylbenzo[b]thiophene-2-sulfonyl chloride was added to a solution containing 92 mg (0.4 mmol) of 3-(1-methyl-piperidin-4-yl)-1H-indazol-5-yl-amine and 120 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 50 mg (26 %) of the desired product.

¹H-NMR (300 MHz, DMSO-d₆): δ 1.61 (m, 4H); 1.91 (m, 2H); 2.18 (s, 3H); 2.26 (m, 3H); 2.72 (m, 3H); 7.05 (d, 1 H, J = 8.8 Hz); 7.17 (s, 1H); 7.34 (d, 1H, J = 8.8 Hz); 7.52 (d, 1H, J = 8.4 Hz); 7.91 (s, 1 H); 8.00 (d, 1H, J = 8.6 Hz); 12.64 (s, 1H)

### 5. naphthalene-1-sulfonic acid [3-(1-methyl-piperidin-4-yl)-1H-indazol-5-yl]-amide

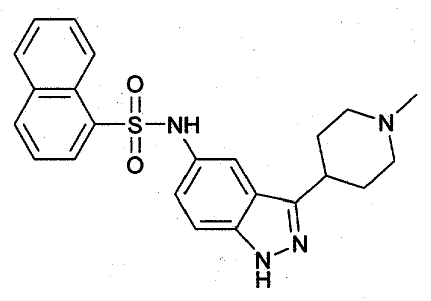

75 mg (0.33 mmol) Naphthalene-1-sulfonyl chloride was added to a solution containing 70 mg (0.3 mmol) of 3-(1-methyl-piperidin-4-yl)-1H-indazol-5-yl-amine and 90 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 60 mg (48 %) of the desired product.

¹H-NMR (300 MHz, DMSO-d₆): δ 1.62 (m, 4H); 1.97 (m, 2H); 2.21 (s, 3H); 2.68 (m, 1 H); 2.81 (d, 1H, J = 10.8 Hz); 6.90 (dd, 1 H, J = 8.6 Hz, J' = 1.6 Hz); 7.11 (s, 1 H); 7.20 (d, 1 H, J = 8.8 Hz); 7.48 (d, 1H, J = 7.9 Hz); 7.62 - 7.71 (m, 2H); 8.03 (d, 2H, J = 7.5 Hz); 8.13 (d, 1 H, J = 8.6 Hz); 8.74 (m, 1H); 12.53 (s, 1 H)

### 6. 4-phenylbenzene-4-sulfonic acid [3-(1-methyl-piperidin-4-yl)-1H-indazol-5-yl]-amide

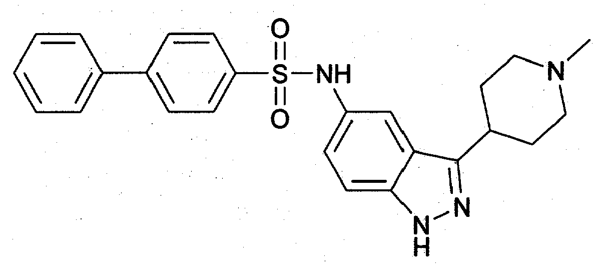

160 mg (0.66 mmol) 4-Phenylbenzene-sulfonyl chloride was added to a solution containing 140 mg (0.6 mmol) of 3-(1-methyl-piperidin-4-yl)-1H-indazol-5-yl-amine and 180 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 135 mg (51 %) of the desired product.

¹H-NMR (300 MHz, CD₃OD): δ 1.91 - 2.06 (m, 4H); 2.23 (m, 2H); 2.37 (s, 3H); 2.97 (m, 3H); 7.29 (m, 1 H); 7.34 (m, 1 H); 7.44 - 7.58 (m, 4H); 7.72 (m, 2H); 7.80 (s, 4H)

### 7. N-[3-(1-methyl-piperidin-4-yl)-1H-indazol-5-yl]-4-phenoxy-benzenesulfonamide

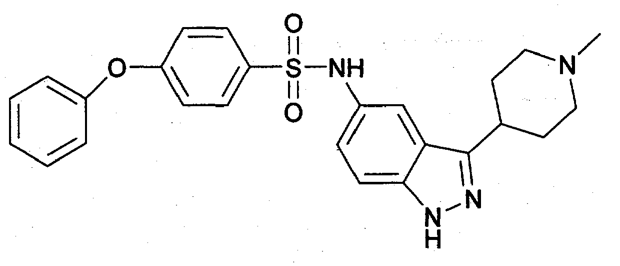

166 mg (0.66 mmol) 4-Phenoxybenzene-sulfonyl chloride was added to a solution containing 140 mg (0.6 mmol) of 3-(1-methyl-piperidin-4-yl)-1H-indazol-5-yl-amine and 180 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 135 mg (51 %) of the desired product.

¹H-NMR (300 MHz, CD₃OD): δ 2.02 - 2.14 (m, 4H); 2.36 (m, 2H); 2.46 (s, 3H); 3.03 - 3.16 (m, 3H); 7.04 (m, 2H); 7.09 (m, 2H); 7.23 (dd, 1 H, J = 9.0 Hz, J' = 1.8 Hz); 7.30 (t, 1H, J = 7.3 Hz); 7.43 - 7.63 (m, 4H); 7.72 (d, 2H, J = 8.6 Hz)

### 8. N-[3-(1-methyl-piperidin-4-yl)-1H-indazol-5-yl]-benzenesulfonamide

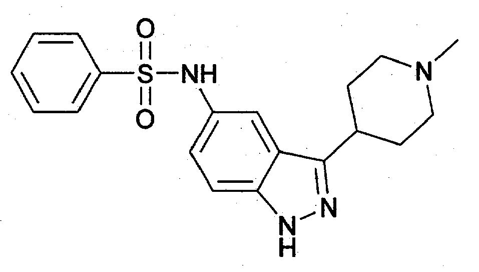

139 mg (0.79 mmol) Benzene sulfonyl chloride was added to a solution containing 165 mg (0.72 mmol) of 3-(1-methyl-piperidin-4-yl)-1H-indazol-5-yl-amine and 216 mg N-diisopropylethylamine in 3 ml dimethylformamide. The reaction mixture was stirred at room temperature for 3 hours. The solvent was evaporated and the residue was taken up in a saturated aqueous solution of NaHCO₃ and extracted with chloroform. The combined organic phases were dried over Na₂SO₄ and concentrated in vacuo. The residue was purified by column chromatography to yield 180 mg (68 %) of the desired product.

¹H-NMR (300 MHz, CD₃OD): δ 1.97 - 2.13 (m, 4H); 2.32 (m, 2H); 2.45 (s, 3H); 2.99 - 3.14 (m, 3H); 7.20 (m, 1 H); 7.42 (d, 1 H, J = 7.3 Hz); 7.43 (d, 1 H, J = 0.9 Hz); 7.49 - 7.57 (m, 2H); 7.59 - 7.66 (m, 1 H); 7.72 (d, 2H, J = 8.6 Hz)

### 9. N-[1-(2-dimethylamino)ethyl)-2,3-dihydro-1H-indol-6-yl]-6-chloro-imidazo[2,1-b]thiazol-5-sulfonamide

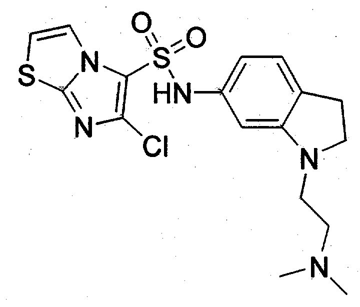

To a solution containing 140 mg (0.68 mmol) of 1-(2-dimethylamino)ethyl)-2,3-dihydro-1H-indol-6-yl-amine and 131 mg N-diisopropylethylamine in 5 ml dimethylformamide was added 192 mg (0.75 mmol) 6-chloro-imidazo[2,1-b]thiazole sulfonyl chloride. The reaction mixture was stirred at ambient temperature for 8 hours. The solvent was evaporated and the residue was taken up in a saturated aqueous NaHCO₃ solution and extracted with chloroform. The combined organic phases were dried over Na₂SO₄ and concentrated in vacuo. The residue was purified by column chromatography to yield 160 mg (55 %) of the desired compound.

Pharmacological data:
The binding of the substituted indazolyl sulfonamide or 2,3-dihydro-indolyl sulfonamide compounds to the 5-HT₆ receptor was determined as described above.
The binding results for some of these compounds are given in the following table :

| **Compound according to example:** | Kᵢ (nM) |
|---|---|
| 1 | 72.6 |
| 2 | 16.4 |
| 3 | 145.6 |
| 4 | 121.5 |
| 9 | 15.8 |

## Claims

1. A substituted indazolyl sulfonamide or 2,3-dihydro-indolyl sulfonamide compound of general formula I, 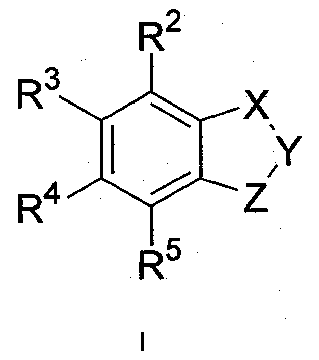 wherein
X-Y from left to right represents CR¹=N and Z is N[(CH₂)ₙR⁶]
or
X-Y from left to right represents CR⁷=N, Z is NH, R⁷ represents the following moiety 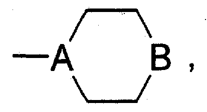 A represents CH or N and B represents NR⁸, 0 or S;
X-Y from left to right represents C[(CH₂)ₙR⁹]=N and Z is NR¹⁰
or
X-Y- represents CH₂-CH₂ and Z is N[(CH₂)ₙR¹¹];
n is 0, 1, 2, 3 or 4;
R¹ represents a hydrogen atom; NO₂; -NH₂; -SH; -OH; -CN; -C(=O)-R¹²; -OR¹³; -SR¹⁴; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -SH, -O-CH₃, -O-C₂H₅, - NO₂, -CN and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, - C(=O)-OH, -C(=O)-O-C₁₋₅-alkyl, -O-C(=O)-C₁₋₅-alkyl, F, Cl, Br, I, -CN, -OCF₃, - SCF₃, -OH, -SH, -NH₂, -NH(C₁₋₅-alkyl), -N(C₁₋₅-alkyl)₂, -NH-C(=O)-C₁₋₅-alkyl, - N(C₁₋₅-alkyl)-C(=O)-C₁₋₅-alkyl, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s);
R², R³, R⁴ and R⁵, independent from one another, each represent a hydrogen atom; -NO₂; -NH₂; -SH; -OH; -CN; -C(=O)-H; -C(=O)-R¹²; -OR¹³; -SR¹⁴; - N(R¹⁵)-S(=O)₂-R¹⁶; -NH-R¹⁷; -NR¹⁸R¹⁹; F; Cl, Br; I; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O=C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of - CF₃, C₁₋₅-alkyl, -O-C₁₋₅-alkyl, -S-C₁₋₅-alkyl, -C(=O)-OH, -C(=O)-O-C₁₋₅-alkyl, -0-C(=O)-C₁₋₅-alkyl, F, Cl, Br, I, -CN, -OCF₃, -SCF₃, -OH, -SH, -NH₂, -NH(C₁₋₅₋alkyl), -N(C₁₋₅-alkyl)₂, -NH-C(=O)-C₁₋₅-alkyl, -N(C₁₋₅-alkyl)-C(=O)-C₁₋₅-alkyl, - NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene group and wherein the heteroaryl radical contains 1, 2 or 3 heteroatom(s) independently selected from the group consisting of nitrogen, oxygen and sulfur as ring member(s); with the proviso that at least one of the substituents R², R³, R⁴ and R⁵ represents a -N(R¹⁵)-S(=O)₂-R¹⁶ moiety;
R⁶, R⁹ and R¹¹, independent from one another, each represent a -NR²⁰R²¹ radical
or
a saturated or unsaturated 3-, 4-, 5-, 6-, 5- or 8-membered cycloaliphatic radical, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, l,-CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) as ring member(s) independently selected from the group consisting of nitrogen, oxygen and sulfur; R⁸ represents -C(=O)-R²²; a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅₋alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, -NH₂, -SH, -O-CH₃, -O=C₂H₅, -NO₂, -CN, -NH-CH₃ and -S-CH₃ and which may be condensed with a saturated or unsaturated mono- or bicyclic ring system which may be substituted with 1, 2 or 3 substituent(s) independently selected from the group consisting of C₁₋₅-alkyl, -O-C₁₋₅-alkyl, - S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and wherein the heteroaryl radical contains 1, 2 or 3 heteroatom(s) independently selected from the group consisting of nitrogen, oxygen and sulfur as ring member(s);
R¹⁰ represents a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a -S(=O)₂-R²³ moiety;
R¹², R¹³, R¹⁴, R¹⁷, R¹⁸ and R¹⁹, independent from one another, each represent a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, - NH-CH₃ and -S-CH₃; a saturated or unsaturated 3-, 4-, 5-, 6-, 5- or 8-membered cycloaliphatic radical, which may be substituted with 1, 2 or 3 substituent(s) independently selected from the group consisting of C₁₋₅-alkyl, - O-C₁₋₅-alkyl, -S-C₁₋₅-alkyl, oxo (=O), thioxo (=S), -C(=O)-OH, -C(=O)-O-C₁₋₅₋alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be bonded via a linear or branched C₁₋₆ alkylene group; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH,-NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulfur as ring member(s);
R¹⁵ represents a hydrogen atom; a linear or branched, saturated or unsaturated C₁₋₁₀ aliphatic radical which may be substituted with 1, 2 or 3 substituent(s) independently selected from the group consisting of F, Cl, Br, - OH, -NH₂, -SH, -O-CH₃, -O-C₂H₅, -NO₂, -CN, -NH-CH₃ and -S-CH₃; or a - S(=O)₂-R²⁴ moiety;
R¹⁶ and R²⁴, independent from one another, each represent a 5- to 14-membered aryl or heteroaryl radical, which may be substituted with 1, 2 or 3 substituent(s) independently selected from the group consisting of -CF₃, C₁₋₅₋alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), - N(C₁₋₅-alkyl)₂, -NH-C(=O)-C₁₋₅-alkyl, -N(C₁₋₅-alkyl)-C(=O)-C₁₋₅-alkyl, -NO₂, - CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), - C(=O)-OH, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, - OH, -NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
R²⁰ and R²¹, independent from one another, each represent a hydrogen atom; or a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃;
or
R²⁰ and R²¹ together with the bridging nitrogen form a saturated, unsaturated or aromatic 3- to 9-membered heterocyclic ring which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl and which may contain 1, 2 or 3 additional heteroatom(s) independently selected from the group consisting of nitrogen, oxygen and sulfur as a ring member(s) and which may be condensed with an unsaturated or saturated mono- or bicyclic ring system, which may be substituted with 1, 2 or 3 substituent(s) independently selected from the group consisting of C₁₋₅₋alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, CFH₂, -C(=O)-NH₂; -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur;
R²² represents a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃; or a 5- to 14-membered aryl or heteroaryl radical, which may be substituted with 1, 2 or 3 substituent(s) independently selected from the group consisting of -CF₃, C₁₋₅-alkyl, -O-C₁₋₅₋alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, -NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂,-C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s)
and
R²³ represents a linear or branched, saturated or unsaturated C₁₋₁₀ aliphatic radical which may be substituted with 1, 2 or 3 substituent(s) independently selected from the group consisting of F, Cl, Br, -OH, -NH₂, -SH, -O-CH₃, -O-C₂H₅, -N02, -CN, -NH-CH₃ and -S-CH₃; a saturated or unsaturated 3-, 4-, 5-, 6-, 5- or 8-membered cycloaliphatic radical, which may be substituted with 1, 2 or 3 substituent(s) independently selected from the group consisting of C₁₋₅₋alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl; Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl and which may optionally contain 1, 2 or 3 heteroatom(s) independently selected from the group consisting of nitrogen, oxygen and sulfur as ring member(s) and which may be bonded via a linear or branched C₁₋₆ alkylene group; or a 5- to 14-membered aryl or heteroaryl radical, which may be substituted with 1, 2 or 3 substituent(s) independently selected from the group consisting of -CF₃, C₁₋₅-alkyl, -O-C₁₋₅-alkyl, -S-C₁₋₅-alkyl, -C(=O)-OH, -C(=O)-O-C₁₋₅-alkyl, -O-C(=O)-C₁₋₅-alkyl, F, Cl, Br, I, -CN, -OCF₃, -SCF₃, -OH, -SH, - NH₂, -NH(C₁₋₅-alkyl), -N(C₁₋₅-alkyl)₂, -NH-C(=O)-C₁₋₅-alkyl, -N(C₁₋₅-alkyl)-C(=O)-C₁₋₅-alkyl, -NO₂, -CHO, -CF₂H, -CFH₂, -C(=O)-NH₂, -C(=O)-NH(C₁₋₅₋alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and which may be condensed with a saturated or unsaturated mono- or bicyclic ring system, which may be substituted with 1, 2 or 3 substituent(s) independently selected from the group consisting of C₁₋₅-alkyl, -O-C₁₋₅-alkyl, -S-C₁₋₅-alkyl, oxo (=O), thioxo (=S), -C(=O)-OH, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl and whereby the rings of the ring system are 5- 6- or 7-membered and may contain 1, 2 or 3 heteroatom(s) independently selected from the group consisting of nitrogen, oxygen and sulfur and which may be bonded via a linear or branched C₁₋₆ alkylene, C₂₋₆ alkenylene or C₂₋₆ alkinylene group which may be substituted with 1, 2 or 3 substituent(s) independently selected from the group consisting of F, Cl, Br, -OH, -NH₂, -SH, -O-CH₃, -O-C₂H₅, -NO₂, -CN, -NH-CH₃ and -S-CH₃ and wherein the heteroaryl radical contains 1, 2 or 3 heteroatom(s) independently selected from the group consisting of nitrogen, oxygen and sulfur as ring member(s);
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

2. A compound according to claim 1, **characterized in that**
X-Y from left to right represents CR¹=N and Z is N[(CH₂)ₙR⁶]
or
X-Y from left to right represents CR⁷=N, Z is NH, R⁷ represents the following moiety 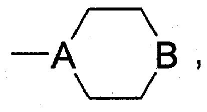 and A represents CH and B represents NR⁸
or
A represents N and B represents NR⁸
or
A represents N and B represents 0
or
A represents N and B represents S
or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1, 2, 3 or 4;
R¹ represents a hydrogen atom; NO₂; -NH₂; -SH; -OH; -CN; -C(=O)-R¹²; -OR¹³; -SR¹⁴; F; Cl, Br; I; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -SH, -O-CH₃, -O-C₂H₅, -NO₂, -CN and -S-CH₃; or an aryl or heteroaryl radical selected from the group-consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl; benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, - O-C(=O)-C(CH₃)₃, F, Cl, Br, I, -CN, -OCF₃, -SCF₃, -OH, -SH, -NH₂, -NH-CH₃, - NH-C₂H₅, -NH-CH(CH₃)₂, -NH-C(CH₃)₃, -N(CH₃)₂, -N(C₂H₅)₂, -N(CH(CH₃)₂)₂, - N0₂, -CHO, -CF₂H, -CFH₂, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
R², R³, R⁴ and R⁵, independent from one another, each represent a hydrogen atom; -NO₂; -NH₂; -SH; -OH; -CN; -C(=O)-H; -C(=O)-R¹²; -OR¹³; -SR¹⁴; - N(R¹⁵)-S(=O)₂-R¹⁶; -NH-R¹⁷; -NR¹⁸R¹⁹; F; Cl; Br; I; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, - OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃,-C(=O)-O-CH(CH₃)₂, -C(=O)-O-C(CH₃)₃, -O-C(=O)-CH₃, -O-C(=O)-CH₂-CH₃, - O-C(=O)-CH(CH₃)₂, -O-C(=O)-C(CH₃)₃, F, Cl, Br, I, -CN, -OCF₃, -SCF₃, -OH, - SH, -NH₂, -NH-CH₃, -NH-C₂H₅, -NH-CH(CH₃)₂, -NH-C(CH₃)₃, -N(CH₃)₂, - N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂-phenyl, cyclopropyl, cyclobutyl, cyclopentyl, cyclohexyl, phenyl, phenoxy and benzyl;
with the proviso that at least one of the substituents R², R³, R⁴ and R⁵ represents a -N(R¹⁵)-S(=O)₂-R¹⁶ moiety;
R⁶, R⁹ and R¹¹, independent from one another, each represent a -NR²⁰R²¹ radical
or
a (hetero)cycloaliphatic radical selected from the group consisting of 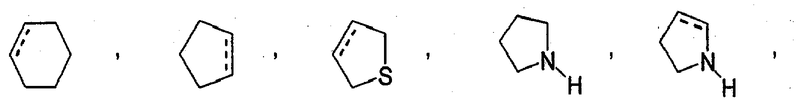 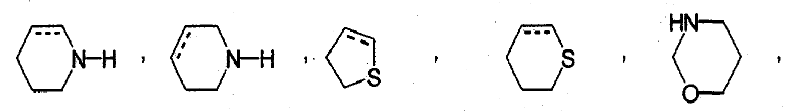 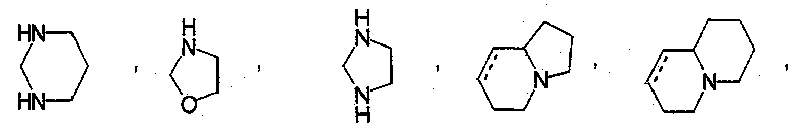 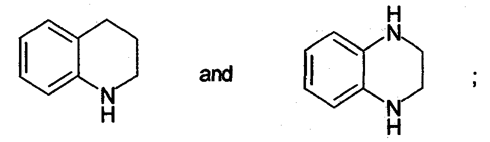 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃,-C(=O)-O-CH(CH₃)₂, -C(=O)-O-C(CH₃)₃, F, Cl, Br, I, -CN, -OCF₃, -SCF₃, -OH, - SH, -NH₂, -NH-CH₃, -NH-C₂H₅, -NH-CH(CH₃)₂, -NH-C(CH₃)₃, -N(CH₃)₂, - N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅ and -S(=O)₂-phenyl in any position including the -NH groups and is not bonded via a nitrogen atom and, if present, the dotted line represents an optional chemical bond;
R⁸ represents -C(=O)-R²²; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -0-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, - O-C(=O)-C(CH₃)₃, F, Cl, Br, I, -CN, -OCF₃, -SCF₃, -OH, -SH, -NH₂, -NH-CH₃, - NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
R¹⁰ represents an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃ or a -S(=O)₂-R²³ moiety;
R¹², R¹³, R¹⁴, R¹⁷, R¹⁸ and R¹⁹, independent from one another, each represent an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -N02, -CN and -S-CH₃; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, which may be bonded via a -(CH₂)_{1, 2 or 3-}group and which may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-CH₂-CH₃, -C(=O)-O-CH(CH₃)₂, -C(=O)-O-C(CH₃)₃, - O-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, - CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅ and - S(=O)₂-phenyl; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which maybe bonded via a -(CH₂)_{1, 2 or 3}- group and which maybe substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, - O-C(=O)-C(CH₃)₃, F, Cl, Br, I, -CN, -OCF₃, -SCF₃, -OH, -SH, -NH2, -NH-CH₃, - NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
R¹⁵ represents a hydrogen atom; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃; or a -S(=O)₂-R²⁴ moiety;
R¹⁶ and R²⁴, independent from one another, each represent an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, - C(=O)-O-CH(CH₃)₂, -C(=O)-O-C(CH₃)₃, -O-C(=O)-CH₃, -O-C(=O)-CH₂-CH₃, - O-C(=O)-CH(CH₃)₂, -O-C(=O)-C(CH₃)₃; F, Cl, Br, I, -CN, -OCF₃, -SCF₃, -OH, - SH, -NH₂, -NH-CH₃, -NH-C₂H₅, -NH-CH(CH₃)₂, -NH-C(CH₃)₃, -N(CH₃)₂, - N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅, -S(=O)₂-phenyl, cyclopropyl, cyclobutyl, cyclopentyl, cyclohexyl, phenyl, phenoxy and benzyl;
R²⁰ and R²¹, independent from one another, each represent a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃
or
R²⁰ and R²¹ together with the bridging nitrogen atom form a moiety selected from the group consisting of 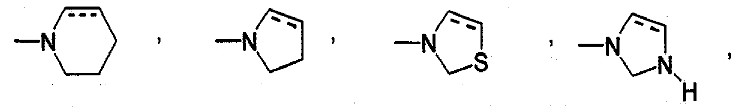 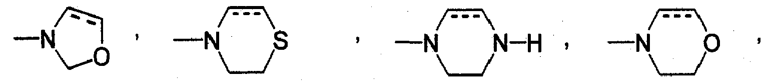 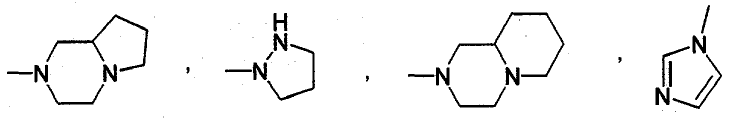 and 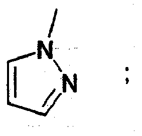 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, - C(=O)-O-CH(CH₃)₂, -C(=O)-O-C(CH₃)₃, -O-C(=O)-CH₃, -O-C(=O)-CH₂-CH₃,-O-C(=O)-CH(CH₃)₂, -O-C(=O)-C(CH₃)₃, F, Cl, Br, I, -CN, -OCF₃, -SCF₃, -OH, - SH, -NH₂, -NH-CH₃, -NH-C₂H₅, -NH-CH(CH₃)₂, -NH-C(CH₃)₃, -N(CH₃)₂, - N(C₂H₅)₂, -N(CH(CH₃)₂)₂, -NO₂, -CHO, -CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅ and -S(=O)₂-phenyl in any position including the -NH groups; and, if present, the dotted line represents an optional chemical bond;
R²² represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -GF₃, methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -0-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-H₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, - O-C(=O)-C(CH₃)₃, F, Cl, Br, I, -CN, -OCF₃, -SCF₃, -OH, -SH, -NH₂, -NH-CH₃, - NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl;
and
R²³ represents alkyl radical selected from the group consisting of methyl, ethyl, n-propyl, isopropyl, n-butyl, sec-butyl, isobutyl and tert-butyl which maybe substituted with 1, 2 or 3 substituent(s) independently selected from the group consisting of F, Cl, Br, -OH, -NH₂, -SH, -O-CH₃, -O-C₂H₅, -NO₂, -CN and -S-CH₃; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, which may be bonded via a -(CH₂)_{1, 2 or 3-}group and which may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, n-pentyl, sec-pentyl, neo-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, - C(=O)-O-CH₃, -C(=O)-O-CH₂-CH₃, -C(=O)-O-CH(CH₃)₂, -C(=O)-O-C(CH₃)₃, - O-C(=O)-CH₃, -O-C(=O)-CH₂-CH₃, -O-C(=O)-CH(CH₃)₂, -O-C(=O)-C(CH₃)₃, F, Cl, Br, I, -CN, -OCF₃, -SCF₃, -OH, -SH, -NH₂, -NH-CH₃, -NH-C₂H₅, -NH-CH(CH₃)₂, -NH-C(CH₃)₃, -N(CH₃)₂, -N(C₂H₅)₂, -N(CH(CH₃)₂)₂, -NO₂, -CHO, - CF₂H, -CFH₂, -C(=O)-NH₂, -C(=O)-NH-CH₃, -C(=O)-NH-C₂H₅, -C(=O)-NH-C(CH₃)₃, -C(=O)-N(CH₃)₂, -C(=O)-N(C₂H₅)₂, -S(=O)₂-CH₃, -S(=O)₂-C₂H₅ and-S(=O)₂-phenyl; or an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, n-pentyl, sec-pentyl, neo-pentyl, -O-CH₃, -O=C₂H₅, -O-CH₂-CH₂-CH₃, -0-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-CH₂-CH₃, -C(=O)-O-CH(CH₃)₂, -C(=O)-O-C(CH₃)₃, -O-C(=O)-CH₃, -O-C(=O)-CH₂-CH₃, -O-C(=O)-CH(CH₃)₂, - O-C(=O)-C(CH₃)₃, F, Cl, Br, I, -CN, -OCF₃, -SCF₃, -OH, -SH, -NH₂, -NH-CH₃, - NH-C₂H₅, -NH-CH(CH₃)₂, -NH-C(CH₃)₃, -N(CH₃)₂, -N(C₂H₅)₂, -N(CH(CH₃)₂)₂, - NO₂, -CHO, -CF₂H, -CFH₂, -C(=O)-NH₂, -C(=O)-NH-CH₃, -C(=O)-NH-C₂H₅, - C(=O)-NH-C(CH₃)₃, -C(=O)-N(CH₃)₂, -C(=O)-N(C₂H₅)₂, -S(=O)₂-CH₃, -S(=O)₂₋C₂H₅, -S(=O)₂-phenyl, cyclopropyl, cyclobutyl, cyclopentyl, cyclohexyl, phenyl, phenoxy and benzyl.

3. A compound according to claim 1 or 2, **characterized in that**
X-Y from left to right represents CR¹=N and Z is N[(CH₂)ₙR⁶]
or
X-Y from left to right represents CR⁷=N, Z is NH, R⁷ represents the following moiety 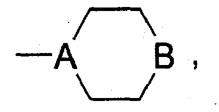 and A represents CH and B represents NR⁸
or
A represents N and B represents NR⁸
or
A represents N and B represents 0
or
A represents N and B represents S
or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1 or 2;
R¹ represents a hydrogen atom; NO₂; -NH₂; -SH; -OH; -CN; F; Cl, Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R², R³, R⁴ and R⁵, independent from one another, each represent a hydrogen atom; -NO₂; -NH₂; -SH; -OH; -CN; -C(=O)-H; -C(=O)-R¹²; -OR¹³; -SR¹⁴; - N(R¹⁵)-S(=O)₂-R¹⁶; -NH-R¹⁷; -NR¹⁸R¹⁹; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting off, Cl, Br, - OH, -NH₂, -SH, -O-CH₃, -O-C₂H₅, -NO₂, -CN and -S-CH₃;
with the proviso that at least one of the substituents R², R³, R⁴ and R⁵ represents a -N(R¹⁵)-S(=O)₂-R¹⁶ moiety;
R⁶, R⁹ and R¹¹, independent from one another, each represent a -NR²⁰R²¹ radical
or
a (hetero)cycloaliphatic radical selected from the group consisting of 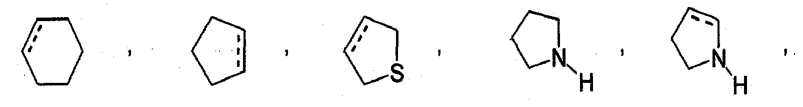 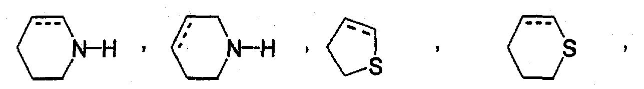 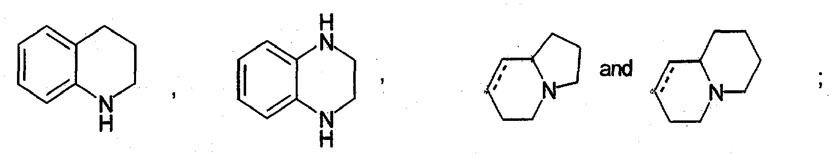 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, -O-CH₃, -O-C₂H₅, -S-CH₃, -S-C₂H₅, -C(=O)-OH, -C(=O)-O-CH₃, F, Cl, Br, I, -CN, -OCF₃, -SCF₃, -OH, -SH, -NH₂, -NH-CH₃, -NH-C₂H₅,-N(CH₃)₂, -N(C₂H₅)₂, -NO₂, -CHO, -CF₂H and -CFH₂ in any position including the -NH groups and is not bonded via a nitrogen atom and, if present, the dotted line represents an optional chemical bond;
R⁸ represents -C(=O)-R²²; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃;
R¹⁰ represents an alkyl radical selected from the group consisting of methyl, ethyl, n-propyl, isopropyl, n-butyl, sec-butyl, isobutyl and tert-butyl or a - S(=O)₂-R²³ moiety;
R¹², R¹³, R¹⁴, R¹⁷, R¹⁸ and R¹⁹, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, -C(=O)-OH, -C(=O)-O-CH₃, - F, Cl, Br, I, -CN, -OCF₃, -SCF₃, -OH, -SH, -NH₂, -NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3}- group and which may be substituted with 1, 2 or 3 substituent(s) independently selected from the group consisting of -CF₃, methyl, ethyl, -O-CH₃, -O-C₂H₅, -O-CH₂₋CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂;
R¹⁵ represents a hydrogen atom; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃; or a -S(=O)₂-R²⁴ moiety;
R¹⁶ and R²⁴, independent from one another, each represent an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH, -NH₂, -NH-CH₃, -NH-C₂H₅, -N(CH₃)₂, -N(C₂H₅)₂, -NO₂ phenyl, phenoxy and benzyl;
R²⁰ and R²¹, independent from one another, each represent a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃
or
R²⁰ and R²¹ together with the bridging nitrogen atom form a moiety selected from the group consisting of 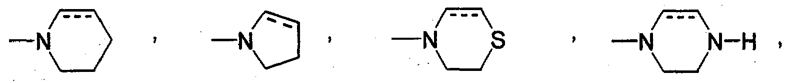 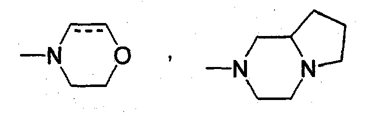 and 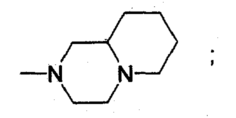 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, - C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂ in any position including the -NH groups; and, if present, the dotted line represents an optional chemical bond;
R²² represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃
and
R²³ represents an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl), pyrrolyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, -O-CH₃, -O-C₂H₅, -O-CH₂-CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, - SH, -NH₂, -NH-CH₃, -NH-C₂H₅, -N(CH₃)₂ and -N(C₂H₅)₂.

4. A compound according to one or more of claims 1 to 3, **characterized in that** one of the substituents R², R³, R⁴ and R⁵ represents a -N(R¹⁵)-S(=O)₂-R¹⁶ moiety.

5. A compound according to claim 4, **characterized in that** one of the substituents R², R³, R⁴ and R⁵ represents a -N(R¹⁵)-S(=O)₂-R¹⁶ moiety and the other substituents R², R³, R⁴ and R⁵ each represent a hydrogen atom.

6. A compound according to claim 4 or 5, **characterized in that** R³ or R⁴ represents a -N(R¹⁵)-S(=O)₂-R¹⁶ moiety and the other substituents R², R³, R⁴ and R⁵ each represent a hydrogen atom.

7. A substituted 1-H-indazolyl sulfonamide compound according to one or more of claims 1 or 3 of general formula la, 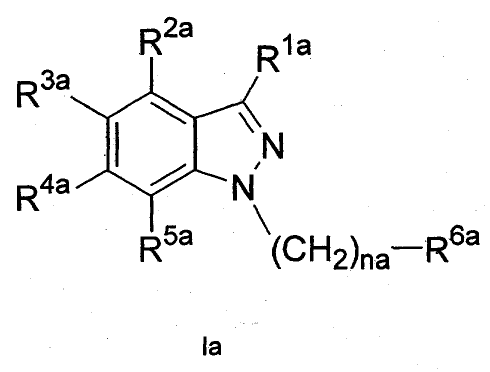 wherein
na is 0, 1 or 2;
R^{1a} represents a hydrogen atom; NO₂; -NH₂; -SH; -OH; -CN; F; Cl, Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R^{2a}, R^{3a}, R^{4a} and R^{5a}; independent from one another, each represent a hydrogen atom; -NO₂; -NH₂; -SH; -OH; -CN; -C(=O)-H; -C(=O)-R^{12a}; -OR^{13a}; - SR^{14a}; -N(R^{15a})-S(=O)₂-R^{16a}; -NH-R^{17a}; -NR^{18a}R^{19a}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, - OH, -NH₂, -SH, -O-CH₃, -O-C₂H₅, -N-O₂, -CN and -S-CH₃;
with the proviso that at least one of the substituents R^{2a}, R^{3a}, R^{4a} and R^{5a} represents a -N(R^{15a})-S(=O)₂-R^{16a} moiety;
R^{6a} represents a -NR^{20a}R^{21a} radical
or
a (hetero)cycloaliphatic radical selected from the group consisting of 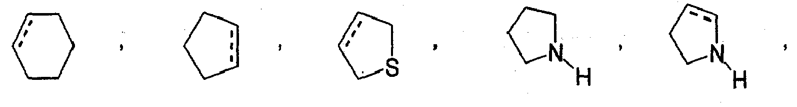 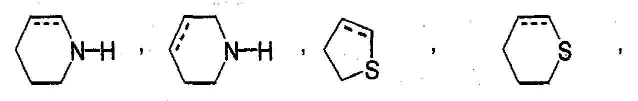 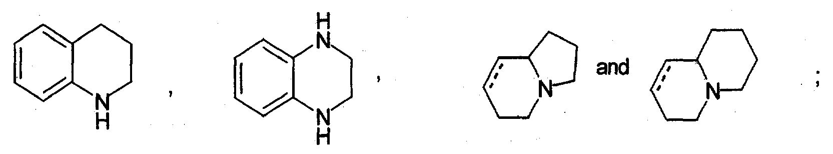 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, -O-CH₃, -O-C₂H₅, -S-CH₃, -S-C₂H₅, -C(=O)-OH, -C(=O)-O-CH₃, F, Cl, Br, I, -CN, -OCF₃, -SCF₃, -OH, -SH, -NH₂, -NH-CH₃, -NH-C₂H₅,-N(CH₃)₂, -N(C₂H₅)₂, -NO₂, -CHO, -CF₂H and -CFH₂ in any position including the -NH groups and is not bonded via a nitrogen atom and, if present, the dotted line represents an optional chemical bond;
R^{12a}, R^{13a}, R^{14a}, R^{17a}, R^{18a} and R^{19a}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyi, piperidinyl, morpholinyl, thiomorpholinyl and piperazinyl, which may be bonded via a -(CH₂)_{1,2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, - O-CH₂-CH₂-CH₃, -S-CH₃, -S-C₂H₅, -C(=O)-OH, -C(=O)-O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, - N(CH₃)₂ and -N(C₂H₅)₂;
R^{15a} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃;
R^{16a} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
and
R^{20a} and R^{21a}, independent from one another, each represent a hydrogen atom; or an alkyl radical selected from the group consisting of methyl, ethyl, n-propyl, isopropyl, n-butyl, sec-butyl, isobutyl and tert-butyl
or
R^{20a} and R^{21a} together with the bridging nitrogen atom form a moiety selected from the group consisting of 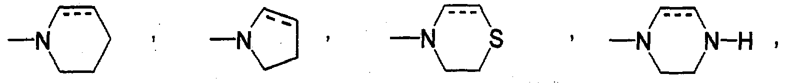 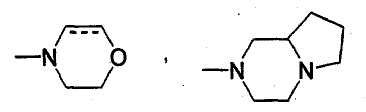 and 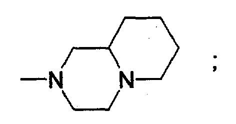 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, - C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂ in any position including the -NH groups; and, if present, the dotted line represents an optional chemical bond;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

8. A compound according to claim 7, **characterized in that**
na is 0, 1 or 2;
R^{1a} represents a hydrogen atom or an alkyl radical selected from the group consisting of methyl, ethyl, n-propyl, isopropyl, n-butyl, sec-butyl, isobutyl and tert-butyl;
R^{2a}, R^{3a}, R^{4a} and R^{5a}, independent from one another, each represent a hydrogen atom; -NO₂; -NH₂; -SH; -OH; -CN; -N(R^{15a})-S(=O)₂-R^{16a}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2a}, R^{3a}, R^{4a} and R^{5a} represents a -N(R^{15a})-S(=O)₂-R^{16a} moiety;
R^{6a} represents a -NR^{20a}R^{21a} radical;
R^{15a} represents a hydrogen atom or an alkyl radical selected from the group consisting of methyl, ethyl, n-propyl, isopropyl, n-butyl, sec-butyl, isobutyl and tert-butyl
R^{16a} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl
and
R^{20a} and R^{21a}, independent from one another, each represent an alkyl radical selected from the group consisting of methyl, ethyl, n-propyl, isopropyl, n-butyl, sec-butyl, isobutyl and tert-butyl.

9. A compound according to claim 7 or 8, **characterized in that**
na is 2;
R^{1a} represents a hydrogen atom or an alkyl radical selected from the group consisting of methyl, ethyl, n-propyl, isopropyl, n-butyl, sec-butyl, isobutyl and tert-butyl;
R^{2a}, R^{3a} and R^{5a}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
R^{4a} represents a -N(R^{15a})-S(=O)₂-R^{16a} moiety;
R^{6a} represents a -NR^{20a}R^{21a} radical;
R^{15a} represents a hydrogen atom;
R^{16a} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, F, Cl, Br, I, phenyl and phenoxy
and
R^{20a} and R^{21a} each represent a methyl radical.

10. A compound according to one or more of claims 1 to 3 and 7 to 9 selected from the group consisting of
[1] N-(1-(2-(Dimethylamino)ethyl)-1H-indazol-6-yl)napthalene-2-sulfonamide and
[2] 5-Chloro-N-(1-(2-(dimethylamino)ethyl)-1H-indazol-6-yl)-3-methylbenzo[b]thiophene-2-sulfonamide;
optionally in form of a physiologically acceptable salt thereof, or a corresponding solvate thereof.

11. A substituted 1-H-indazolyl sulfonamide compound according to one or more of claims 1 to 3 of general formula lb, 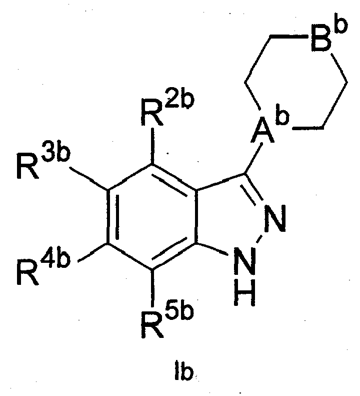 wherein
A^{b} represents CH and B^{b} represents NR^{8b}
or
A^{b} represents N and B^{b} represents NR^{8b}
or
A^{b} represents N and B^{b} represents 0
or
A^{b} represents N and B^{b} represents S;
R^{2b}, R^{3b}, R^{4b} and R^{5b}, independent from one another, each represent a hydrogen atom; -NO₂; -NH₂; -SH; -OH; -CN; -C(=O)-H; -C(=O)-R^{12b}; -OR^{13b}; - SR^{14b}; -N(R^{15b})-S(=O)₂-R^{16b}; -NH-R^{17b}; -NR^{18b}R^{19b}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br,-OH, -NH₂, -SH, -O-CH₃, -O-C₂H₅, -NO₂, -CN and -S-CH₃;
with the proviso that at least one of the substituents R^{2b}, R^{3b}, R^{4b} and R^{5b} represents a -N(R^{15b})-S(=O)₂-R^{16b} moiety;
R^{8b} represents -C(=O)-R^{22b}; an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
R^{12b}, R^{13b}, R^{14b}, R^{17b}, R^{18b} and R^{19b}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, - O-CH₂-CH₂-CH₃, -S-CH₃, -S-C₂H₅, -C(=O)-OH, -C(=O)-O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, - N(CH₃)₂ and -N(C₂H₅)₂;
R^{15b} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃;
R^{16b} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which maybe bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -0-CH₃, -O-C₂H₅, -S-CH₃, -S-C₂H₅, F, Cl, Br, I, -CN, OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
and
R^{22b} represents alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -O-C₂H₅, -NO₂, -CN and -S-CH₃;
optionally in form of a physiologically acceptable salt thereof, or a corresponding solvate thereof.

12. A compound according to claim 11, **characterized in that**
A^{b} represents CH and B^{b} represents NR^{8b}
or
A^{b} represents N and B^{b} represents NR^{8b}
or
A^{b} represents N and B^{b} represents O
or
A^{b} represents N and B^{b} represents S;
R^{2b}, R^{3b}, R^{4b} and R^{5b}, independent from one another, each represent a hydrogen atom; -NO₂; -NH₂; -SH; -OH; -CN; -N(R^{15b})-S(=O)₂-R^{16b}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2b}, R^{3b}, R^{4b} and R^{5b} represents a -N(R^{15b})-S(=O)₂-R^{16b} moiety;
R^{8b} represents an alkyl radical selected from the group consisting of methyl, ethyl, n-propyl, isopropyl, n-butyl, sec-butyl, isobutyl and tert-butyl;
R^{15b} represents a hydrogen atom or an alkyl radical selected from the group consisting of methyl, ethyl, n-propyl, isopropyl, n-butyl, sec-butyl, isobutyl and tert-butyl
and
R^{16b} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.

13. A compound according to claim 11 or 12, **characterized in that**
A^{b} represents CH and B^{b} represents NR^{8b}
R^{2b}, R^{4b} and R^{5b}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
R^{3b} represents a -N(R^{15b})-S(=O)₂-R^{16b} moiety;
R^{8b} represents a methyl radical;
R^{15b} represents a hydrogen atom
and
R^{16b} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, tert-butyl, F, Cl, Br, I, phenyl and phenoxy.

14. A compound according to one or more of claims 1 to 3 and 11 to 13 selected from the group consisting of
[3] Naphthalene-2-sulfonic acid [3-(1-methyl-piperidin-4-yl)-1H-indazol-5-yl]-amide,
[4] 5-Chloro-3-methyl-benzo[b]thiophene-2-sulfonic acid [3-(1-methyl-piperidin-4-yl)-1H-indazol-5-yl]-amide,
[5] Naphthalene-1-sulfonic acid [3-(1-methyl-piperidin-4-yl)-1H-indazol-5-yl]-amide,
[6] 4-Phenylbenzene-4-sulfonic acid [3-(1-methyl-piperidin-4-yl)-1H-indazol-5-yl]-amide,
[7] N-[3-(1-Methyl-piperidin-4-yl)-1H-indazol-5-yl]-4-phenoxy-benzenesulfonamide
and
[8] N-[3-(1-Methyl-piperidin-4-yl)-1H-indazol-5-yl]-benzenesulfonamide;
optionally in form of a physiologically acceptable salt thereof, or a corresponding solvate thereof.

15. A substituted 1-H-indazolyl sulfonamide compound according to one or more of claims 1 to 3 of general formula Ic, 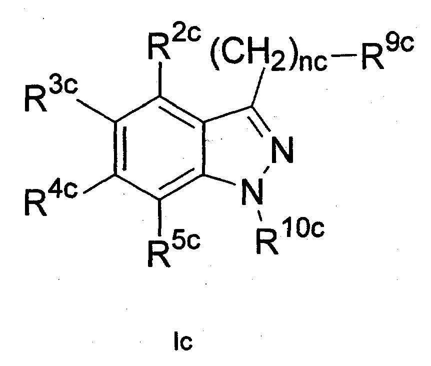 wherein
nc is 0,1 or 2;
R^{2c}, R^{3c}, R^{4c} and R^{5c}, independent from one another, each represent a hydrogen atom; -NO₂; -NH₂; -SH; -OH; -CN; -C(=O)-H; -C(=O)-R^{12c}; -OR^{13c}; - SR^{14C}; -N(R^{15c})-S(=O)₂-R^{16c}; -NH-R^{17c}; -NR^{18c}R^{19c}; F; Cl; Br; I; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, - OH, -NH₂, -SH, -O-CH₃, -O-C₂H₅, -NO₂, -CN and -S-CH₃;
with the proviso that at least one of the substituents R^{2c}, R^{3c}, R^{4c} and R^{5c} represents a -N(R^{15c})-S(=O)₂-R^{16c} moiety;
R^{9c} represents a -NR^{20c}R^{21c} radical
or
a (hetero)cycloaliphatic radical selected from the group consisting of 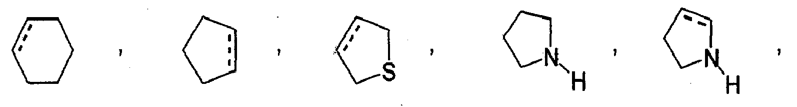 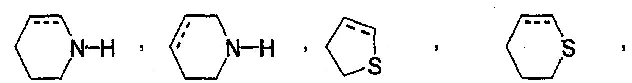 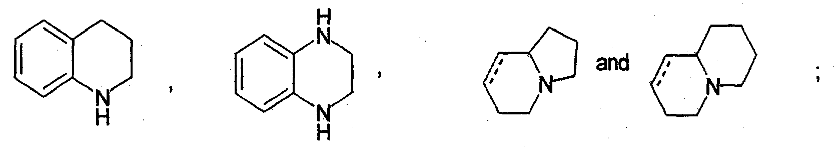 whereby each of these afore mentioned cyclic moieties may be substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, -O-CH₃, -O-C₂H₅, -S-CH₃, -S-C₂H₅, -C(=O)-OH, -C(=O)-O-CH₃, F, Cl, Br, I, -CN, -OCF₃, -SCF₃, -OH, -SH, -NH₂, -NH-CH₃, -NH-C₂H₅,-N(CH₃)₂, -N(C₂H₅)₂, -NO₂, -CHO, -CF₂H and -CFH₂ in any position including the -NH groups and is not bonded via a nitrogen atom and, if present, the dotted line represents an optional chemical bond;
R^{10c} represents an alkyl radical selected from the group consisting of methyl, ethyl, n-propyl, isopropyl, n-butyl, sec-butyl, isobutyl and tert-butyl or a - S(=O)₂-R^{23c} moiety;
R^{12c}, R^{13c}, R^{14c}, R^{17c}, R^{18c} and R^{19c}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, - O-CH₂-CH₂-CH₃, -S-CH₃, -S-C₂H₅, -C(=O)-OH, -C(=O)-O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, - N(CH₃)₂ and -N(C₂H₅)₂;
R^{20c} and R^{21c}, independent from one another, each represent a hydrogen atom; or an alkyl radical selected from the group consisting of methyl, ethyl, n-propyl, isopropyl, n-butyl, sec-butyl, isobutyl and tert-butyl;
or
R^{20c} and R^{21c} together with the bridging nitrogen atom form a moiety selected from the group consisting of 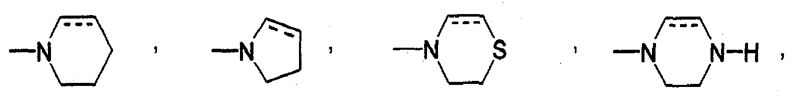 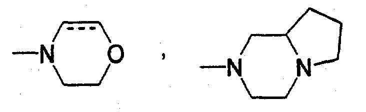 and 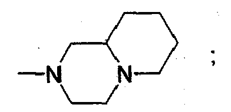 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, - C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂ in any position including the -NH groups; and, if present, the dotted line represents an optional chemical bond
and
R^{23c} represents an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which may be substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl), pyrrolyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which maybe bonded via a -(CH₂)_{1, 2 or 3}- group and which may be substituted with 1, 2 or 3 substituent(s) independently selected from the group consisting of -CF₃, methyl, ethyl, -O-CH₃, -O-C₂H₅, -O-CH₂-CH₂-CH₃, -S-CH₃, -S-C₂H₅, -C(=O)-OH, -C(=O)-O-CH₃, -C(=O)-O-CH₂-CH₃, F, Cl, Br, I, -CN, -OCF₃, -SCF₃, -OH, - SH, -NH₂, -NH-CH₃, -NH-C₂H₅, -N(CH₃)₂ and -N(C₂H₅)₂;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

16. A compound according to claim 15, **characterized in that**
nc is 0, 1 or 2;
R^{2c}, R^{3c}, R^{4c} and R^{5c}, independent from one another, each represent a hydrogen atom; -NO₂; -NH₂; -SH; -OH; -CN; -N(R^{15c})-S(=O)₂-R^{16c}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2c}, R^{3c}, R^{4c} and R^{5c} represents a -N(R^{15c})-S(=O)₂-R^{16c} moiety;
R^{9c} represents a -NR^{20c}R^{21c} radical;
R^{10c} represents an alkyl radical selected from the group consisting of methyl, ethyl, n-propyl, isopropyl, n-butyl, sec-butyl, isobutyl and tert-butyl or a - S(=O)₂-R^{23c} moiety;
R^{15c} represents a hydrogen atom or an alkyl radical selected from the group consisting of methyl, ethyl, n-propyl, isopropyl, n-butyl, sec-butyl, isobutyl and tert-butyl
R^{16c} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.
R^{20c} and R^{21c}, independent from one another, each represent an alkyl radical selected from the group consisting of methyl, ethyl, n-propyl, isopropyl, n-butyl, sec-butyl, isobutyl and tert-butyl;
or
R^{20c} and R^{21c} together with the bridging nitrogen atom form an unsubstituted moiety selected from the group consisting of 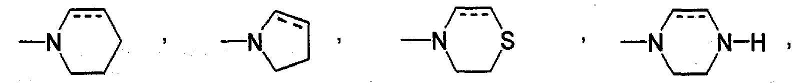 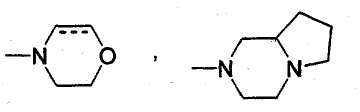 and 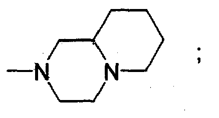 wherein, if present, the dotted line represents an optional chemical bond;
and
R^{23c} represents an alkyl radical selected from the group consisting of methyl, ethyl, n-propyl, isopropyl, n-butyl, sec-butyl, isobutyl and tert-butyl or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.

17. A compound according to claim 15 or 16, **characterized in that**
nc is 0, 1 or 2;
R^{2c}, R^{3c}, R^{4c} and R^{5c}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
with the proviso that either R^{3c} or R^{4c} represents a -N(R^{15c})-S(=O)₂-R^{16c} moiety;
R^{9c} represents a -NR^{20c}R^{21c} radical;
R^{10c} represents an alkyl radical selected from the group consisting of methyl, ethyl, n-propyl, isopropyl, n-butyl, sec-butyl, isobutyl and tert-butyl or a - S(=O)₂-R^{23c} moiety;
R^{15c} represents a hydrogen atom;
R^{16c} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, tert-butyl, F, Cl, Br and I;
R^{20c} and R^{21c}, independent from one another, each represent an alkyl radical selected from the group consisting of methyl, ethyl, n-propyl, isopropyl, n-butyl, sec-butyl, isobutyl and tert-butyl
and
R^{23c} represents an alkyl radical selected from the group consisting of methyl, ethyl, n-propyl, isopropyl, n-butyl, sec-butyl, isobutyl and tert-butyl or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.

18. A substituted 2,3-dihydro-indolyl compound according to one or more of claims 1 to 3 of general formula Id, 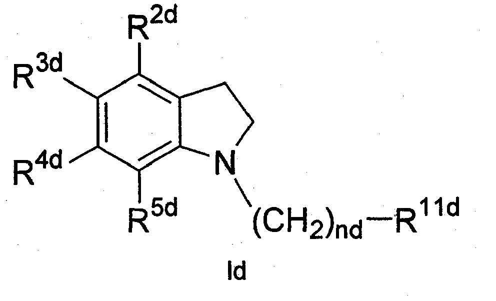 wherein
nd is 0, 1 or 2;
R^{2d}, R^{3d}, R^{4d} and R^{5d}, independent from one another, each represent a hydrogen atom; -NO₂; -NH₂; -SH; -OH; -CN; -C(=O)-H; -C(=O)-R^{12d}; -OR^{13d}; - SR^{14d}; -N(R^{15d})-S(=O)₂-R^{16d}; -NH-R^{17d}; -NR^{18d}R^{19d}; F; Cl; Br; I; or an alkyl radical selected from the group consisting of methyl, ethyl, n-propyl, isopropyl, n-butyl, sec-butyl, isobutyl and tert-butyl which maybe substituted with 1, 2 or 3 substituent(s) independently selected from the group consisting of F, Cl, Br, - OH, -NH₂, -SH, -O-CH₃, -O-C₂H₅, -NO₂, -CN and -S-CH₃;
with the proviso that at least one of the substituents R^{2d}, R^{3d}, R^{4d} and R^{5d} represents a -N(R^{15d})-S(=O)₂-R^{16d} moiety;
R^{11d} represents a -NR^{20d}R^{21d} radical
or
a (hetero)cycloaliphatic radical selected from the group consisting of 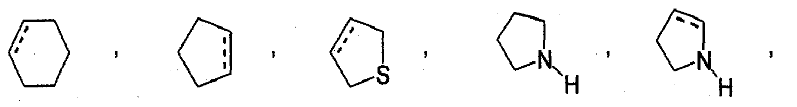 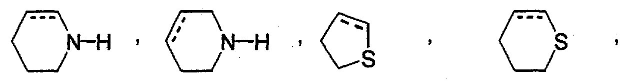 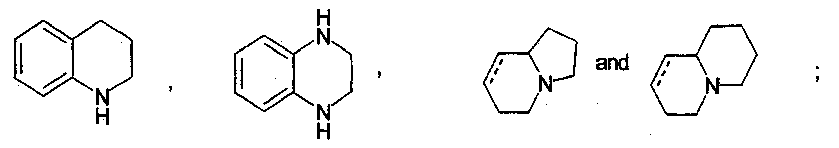 whereby each of these afore mentioned cyclic moieties maybe substituted with 1, 2 or 3 substituent(s) independently selected from the group consisting of oxo (=O), thioxo (=S), methyl, ethyl, n-propyl, isopropyl, n-butyl, sec-butyl, isobutyl, tert-butyl, -O-CH₃, -O-C₂H₅, -S-CH₃, -S-C₂H₅, -C(=O)-OH, -C(=O)-O-CH₃, F, Cl, Br, I, -CN, -OCF₃, -SCF₃, -OH, -SH, -NH₂, -NH-CH₃, -NH-C₂H₅,-N(CH₃)₂, -N(C₂H₅)₂, -NO₂, -CHO, -CF₂H and -CFH₂ in any position including the -NH groups and is not bonded via a nitrogen atom and, if present, the dotted line represents an optional chemical bond;
R^{12d}, R^{13d}, R^{14d}, R^{17d}, R^{18d} and R^{19d}, independent from one another, each represent an alkyl radical selected from the group consisting of methyl, ethyl, n-propyl, isopropyl, n-butyl, sec-butyl, isobutyl and tert-butyl; a (hetero)cycloaliphatic radical selected from the group consisting of cyclopentyl, cyclohexyl, pyrrolidinyl, piperidinyl, morpholinyl, thiomorpholinyl and piperazinyl, which may be bonded via a -(CH₂)_{1, 2 or 3}- group and which maybe substituted with 1, 2 or 3 substituent(s) independently selected from the group consisting of oxo (=O), thioxo (=S), methyl, ethyl, -O-CH₃, -O-C₂H₅, -S-CH₃, - C(=O)-OH, -C(=O)-O-CH₃, - F, Cl, Br, I, -CN, -OCF₃, -SCF₃, -OH, -SH, -NH₂, - NH-CH₃, -NH-C₂H₅, -N(CH₃)₂ and -N(C₂H₅)₂; or an aryl or heteroaryl radical selected from the group consisting of phenyl, naphthyl, pyridinyl, furyl (furanyl), thienyl (thiophenyl) and pyrrolyl, which may be bonded via a -(CH₂)_{1, 2 or 3-}group and which may be substituted with 1, 2 or 3 substituent(s) independently selected from the group consisting of -CF₃, methyl, ethyl, -O-CH₃, -O-C₂H₅, - O-CH₂-CH₂-CH₃, -S-CH₃, -S-C₂H₅, -C(=O)-OH, -C(=O)-O-CH₃, -C(=O)-O-CH₂₋CH₃, F, Cl, Br, I, -CN, -OCF₃, -SCF₃, -OH, -SH, -NH₂, -NH-CH₃, -NH-C₂H₅, - N(CH₃)₂ and -N(C₂H₅)₂;
R^{15d} represents a hydrogen atom; or an alkyl radical selected from the group consisting of methyl, ethyl, n-propyl, isopropyl, n-butyl, sec-butyl, isobutyl and tert-butyl which may be substituted with 1, 2 or 3 substituent(s) independently selected from the group consisting of F, Cl, Br, -OH, -NH₂, -SH, -O-CH₃, -0-C₂H₅, -NO₂, -CN and -S-CH₃;
R^{16d} represents an aryl or heteroaryl radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, which may be bonded via a -(CH₂)_{1, 2 or 3}- group and which may be substituted with 1, 2 or 3 substituent(s) independently selected from the group consisting of -CF₃, methyl, ethyl, n-propyl, isopropyl, n-butyl, sec-butyl, isobutyl, tert-butyl, -O-CH₃, -O-C₂H₅, -S-CH₃, -S-C₂H₅, F, Cl, Br, I, -CN, -OCF₃, -SCF₃, -OH, -SH, - NH₂, -NH-CH₃, -NH-C₂H₅, -N(CH₃)₂, -N(C₂H₅)₂, -NO₂, phenyl, phenoxy and benzyl;
and
R^{20d} and R^{21d}, independent from one another, each represent a hydrogen atom; or an alkyl radical selected from the group consisting of methyl, ethyl, n-propyl, isopropyl, n-butyl, sec-butyl, isobutyl and tert-butyl;
or
R^{20d} and R^{21d} together with the bridging nitrogen atom form a moiety selected from the group consisting of 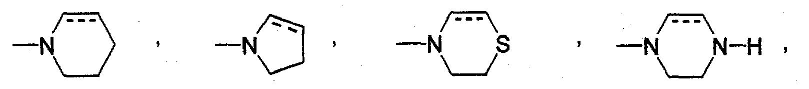 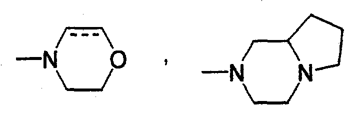 and 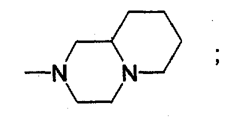 whereby each of these afore mentioned cyclic moieties may be substituted with 1, 2 or 3 substituent(s) independently selected from the group consisting of oxo (=O), thioxo (=S), methyl, ethyl, -O-CH₃, -O-C₂H₅, -S-CH₃, -S-C₂H₅, - C(=O)-OH, -C(=O)-O-CH₃, -C(=O)-O-CH₂-CH₃, F, Cl, Br, I, -CN, -OCF₃, -SCF₃, -OH, -SH, -NH₂, -NH-CH₃, -NH-C₂H₅, -N(CH₃)₂ and -N(C₂H₅)₂ in any position including the -NH groups; and, if present, the dotted line represents an optional chemical bond;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

19. A compound according to claim 18, **characterized in that**
nd is 0, 1 or 2;
R^{2d}, R^{3d}, R^{4d} and R^{5d}, independent from one another, each represent a hydrogen atom; -NO₂; -NH₂; -SH; -OH; -CN; -N(R^{15d})-S(=O)₂-R^{16d}; F; Cl; Br; I; or an alkyl radical selected from the group consisting of methyl, ethyl, n-propyl, isopropyl, n-butyl, sec-butyl, isobutyl and tert-butyl;
with the proviso that at least one of the substituents R^{2d}, R^{3d}, R^{4d} and R^{5d} represents a -N(R^{15d})-S(-O)₂-R^{16d} moiety;
R^{11d} represents a -NR^{20d}R^{21d} radical;
R^{15d} represents a hydrogen atom or an alkyl radical selected from the group consisting of methyl, ethyl, n-propyl, isopropyl, n-butyl, sec-butyl, isobutyl and tert-butyl;
R^{16d} represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl
and
R^{20d} and R^{21d}, independent from one another, each represent an alkyl radical selected from the group consisting of methyl, ethyl, n-propyl, isopropyl, n-butyl, sec-butyl, isobutyl and tert-butyl.

20. A compound according to claim 18 or 19, **characterized in that**
nd is 2;
R^{2a}, R^{3a} and R^{5d}, independent from one another, each represent a hydrogen atom; F; Cl; Br; I; or an alkyl radical selected from the group consisting of methyl, ethyl, n-propyl, isopropyl, n-butyl, sec-butyl, isobutyl and tert-butyl;
R^{4d} represents a -N(R^{15d})-S(=O)₂-R^{16d} moiety;
R^{11d} represents a -NR^{20d}R^{21d} radical;
R^{15d} represents a hydrogen atom;
R^{16d} represents an imidazo[2,1-b]thiazolyl radical, which may be substituted with 1, 2 or 3 substituent(s) independently selected from the group consisting of F, Cl, Br and I
and
R^{20d} and R^{21d} each represent a methyl radical.

21. The following compound according to one or more of claims 1 to 3 and 18 to 20
[9] N-[1-(2-Dimethylamino)ethyl)-2,3-dihydro-1H-indol-6-yl]-6-chloro-imidazo[2,1-b]thiazol-5-sulfonamide;
optionally in form of a physiologically acceptable salt thereof, or a corresponding solvate thereof.

22. A compound according to one or more of claims 1 to 3, **characterized in that**
X-Y from left to right represents CR¹=N and Z is N[(CH₂)ₙR⁶]
or
X-Y from left to right represents CR⁷=N, Z is NH, R⁷ represents the following moiety 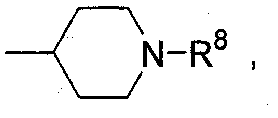 or
X-Y from left to right represents C[(CH₂)ₙR⁹]=N and Z is NR¹⁰
or
X-Y- represents CH₂-CH₂ and Z is N[(CH₂)ₙR¹¹];
n is 0, 1 or 2;
R¹ represents a hydrogen atom or an alkyl radical selected from the group consisting of methyl, ethyl, n-propyl, isopropyl, n-butyl, sec-butyl, isobutyl and tert-butyl;
R², R³, R⁴ and R⁵, independent from one another, each represent a hydrogen atom; F; Cl; Br; I; an -N(R¹⁵)-S(=O)₂-R¹⁶- moiety, or an alkyl radical selected from the group consisting of methyl, ethyl, n-propyl, isopropyl, n-butyl, sec-butyl, isobutyl and tert-butyl;
with the proviso that either R³ or R⁴ represents a -N(R¹⁵)-S(=O)₂-R¹⁶ moiety;
R⁶ represents a -NR²⁰R²¹ radical;
R⁸ represents an alkyl radical selected from the group consisting of methyl, ethyl, n-propyl, isopropyl, n-butyl, sec-butyl, isobutyl and tert-butyl;
R⁹ represents a -NR²⁰R²¹ radical;
R¹⁰ represents an alkyl radical selected from the group consisting of methyl, ethyl, n-propyl, isopropyl, n-butyl, sec-butyl, isobutyl and tert-butyl or a - S(=O)₂-R²³ moiety;
R¹¹ represents a -NR²⁰R²¹ radical;
R¹⁵ represents a hydrogen atom;
R¹⁶ represents an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, F, Cl, Br and I;
R²⁰ and R²¹, independent from one another, each represent an alkyl radical selected from the group consisting of methyl, ethyl, n-propyl, isopropyl, n-butyl, sec-butyl, isobutyl and tert-butyl
and
R²³ represents an alkyl radical selected from the group consisting of methyl, ethyl, n-propyl, isopropyl, n-butyl, sec-butyl, isobutyl and tert-butyl or an aryl or heteroaryl radical selected from the group consisting of phenyl, naphthyl, benzo[b]furanyl, benzo[b]thiophenyl and imidazo[2,1-b]thiazolyl, which may be substituted with 1, 2 or 3 substituent(s) independently selected from the group consisting of methyl, ethyl, n-propyl, isopropyl, n-butyl, sec-butyl, isobutyl, tert-butyl, F, Cl, Br, I, -CN, phenyl, phenoxy and benzyl.

23. Process for the preparation of a compound according to one or more of claims 1 to 22, **characterized in that** at least one compound of general formula II, 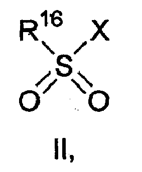 wherein R¹⁶ has the meaning according to one or more of claims 1 to 22 and X represents a leaving group, preferably a halogen atom, more preferably a chlorine atom, is reacted with at least one compound of general formula III, 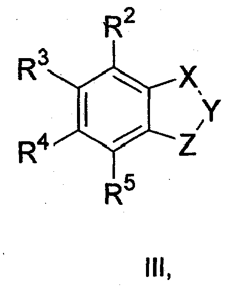 wherein X, Y, Z and R² to R⁵ have the meaning according to one or more of claims 1 to 22 with the proviso that at least one of the substituents R², R³, R⁴ and R⁵, represents a -NH₂ group, in a suitable reaction medium, preferably in the presence of at least one base, to yield a compound of general formula I, wherein X, Y, Z and R² to R⁵ have the meaning according to one or more of claims 1 to 22 with the proviso that at least one of the substituents R², R³, R⁴ and R⁵ represents a -N(H)-S(=O)₂-R¹⁶ group and R¹⁶ has the meaning according to one or more of claims 1 to 22, which is optionally purified and/or isolated,
and optionally said compound of general formula I, wherein X, Y, Z and R² to R⁵ have the meaning according to one or more of claims 1 to 22 with the proviso that at least one of the substituents R², R³, R⁴ and R⁵ represents a - N(H)-S(=O)₂-R¹⁶ group and R¹⁶ has the meaning according to one or more of claims 1 to 22, is reacted with at least one compound of general formula R¹⁵⁻X, wherein R¹⁵ has the meaning according to one or more of claims 1 to 22 and X represents a halogen atom, preferably a chlorine atom, in a suitable reaction medium, in the presence of at least one base, preferably at least one base selected from the group consisting of metal hydroxides, metal carbonates, metal hydrides, metal alcoxides, preferably sodium methoxide or potassium tert-butoxid, and organometallic compounds, preferably n-butyllithium and tert-butyllithium,
or with at least one compound of general formula X-S(=O)₂-R²⁴, wherein R²⁴ has the meaning according to one or more of claims 1 to 22 and X represents a leaving group, preferably a halogen atom, more preferably a chlorine atom, in a suitable reaction medium, preferably in the presence of at least one base,
to yield a compound of general formula I, wherein X, Y, Z and R² to R⁵ have the meaning according to one or more of claims 1 to 22 with the proviso that at least one of the substituents R², R³, R⁴ and R⁵ represents a -N(R¹⁵)-S(=O)₂₋R¹⁶ group and R¹⁵ and R¹⁶ have the meaning according to one or more of claims 1 to 22, which is optionally purified and/or isolated.

24. Medicament comprising at least one compound according to one or more of claims 1 to 22 and optionally at least one physiologically acceptable auxiliary agent.

25. Medicament according to claim 24 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

26. Use of at least one compound according to one or more of claims 1 to 22 for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

27. Use of at least one compound according to one or more of claims 1 to 22 for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder).

28. Use of at least one compound according to one or more of claims 1 to 22 for the manufacture of a medicament for the improvement of cognition (cognitive enhancement) and/or for the improvement of cognitive memory (cognitive memory enhancement).

29. Use of at least one compound according to one or more of claims 1 to 22 for the manufacture of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine.
